# EUROPEAN PATENT SPECIFICATION

(11) **EP 3 177 645 B1**
(45) Date of publication and mention of the grant of the patent: **17.02.2021**
(21) Application number: 15756110.1
(22) Date of filing: 10.08.2015
(51) Int. Cl.: C07K 16/28, C07K 16/32, A61K 39/00

(54) **SUBCUTANEOUSLY ADMINISTERED BISPECIFIC ANTIBODIES FOR USE IN THE TREATMENT OF CANCER**
SUBKUTAN VERABREICHTE BISPEZIFISCHE ANTIKÖRPER ZUR VERWENDUNG IN DER KREBSBEHANDLUNG
ANTICORPS BISPÉCIFIQUES ADMINISTRÉS PAR VOIE SOUS-CUTANÉE DESTINÉS À ÊTRE UTILISÉS DANS LE TRAITEMENT DU CANCER

(30) Priority: 08.08.2014 WO PCT/EP2014/002183
(43) Date of publication of application: 14.06.2017
(73) Proprietor: Lindis Biotech GmbH, 82152 Martinsried (DE)
(72) Inventor: BUHMANN, Raymund, 86911 Diessen am Ammersee (DE); DREYLING, Martin, 82152 Planegg (DE); HIDDEMANN, Wolfgang, 82319 Starnberg (DE); LINDHOFER, Horst, 80639 München (DE)
(74) Representative: Graf von Stosch, Andreas
(86) International application number: PCT/EP2015/001646
(87) International publication number: WO 2016/020065

(56) References cited:
- WO-A1-2014/072277
- WO-A1-2014/072277
- WO-A2-2014/028560
- US-A1- 2014 050 660

## Description

### FIELD OF THE INVENTION

The present invention relates to the use of bispecific antibodies, namely, T-cell redirecting, bispecific antibodies comprising a specificity against a tumor-associated antigen and an Fc region, for the treatment of cancer.

More specifically, the present invention relates to a dosage regime for use in the treatment of cancer wherein a bispecific antibody, in particular a trifunctional antibody, is administered subcutaneously to a patient diagnosed with at least one type of cancer comprising (a) the subcutaneous administration of an initial dose of said antibody; and consecutively (b) subcutaneously administering at least one repeating dose of said antibody; wherein said at least one repeating dose is equal to or exceeds the previous dose by a factor of 1.5 to 3 of the amount administered by the previous dose.

### BACKGROUND OF THE INVENTION

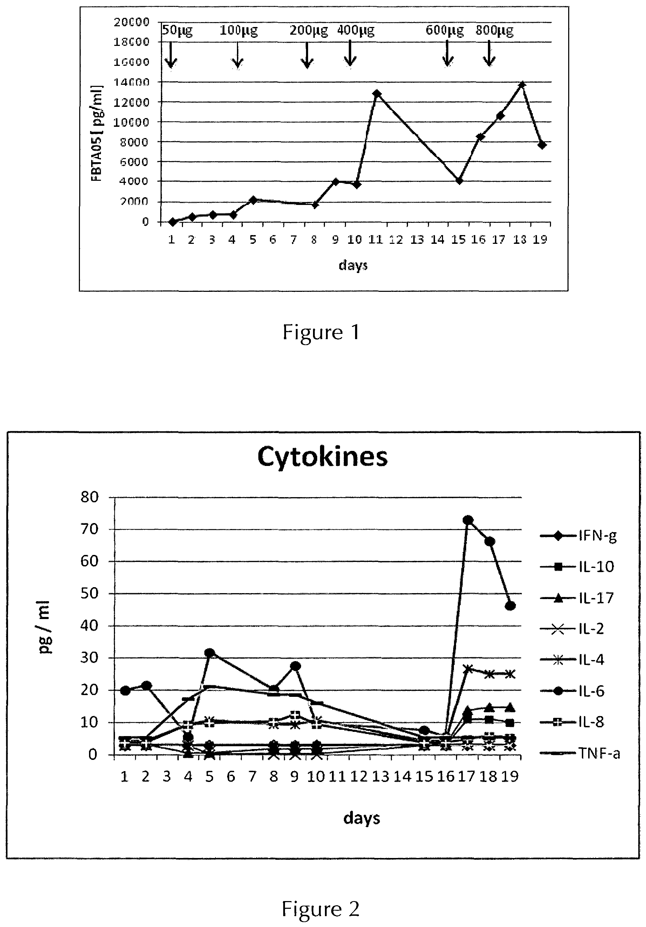

Bispecific antibodies (BiAbs) offer a unique opportunity in cancer immunotherapy by redirecting immune effector cells to kill cancer cells. Bispecific antibodies (BiAbs) are antibody-derived proteins with the ability to bind to two different epitopes on the same or different antigens. Bispecific antibodies (BiAbs) therefore range from antibodies, such as whole IgG-like molecules, to small recombinant formats, such as tandem single chain variable fragment molecules (taFvs), diabodies (Dbs), single chain diabodies (scDbs) and various other derivatives of these (cf. Byrne H. et al. (2013) Trends Biotech, 31 (11): 621-632 with Figure 2 showing various bispecific antibody formats). Several BiAb formats can redirect effector cells against target cells that play key roles in disease processes. For example, WO 2014/028560 discloses a T-cell redirecting complex, which may comprise a bispecifc antibody, for example an anti-CD3 x anti-CD19 antibody, for the treatment of cancer. WO 2014/072277 relates to a formulation for bispecific T-cell engagers (BiTEs), which comprises TRIS and phosphate, and discloses subcutaneous administration of such a formulation containing a BiTE antibody. For example, several BiAb formats can retarget effector cells towards tumor cells and a variety of BiAb constructs were designed to retarget cells of the immune system, for example by binding to and triggering Fc receptors on the surface of effector cells or by binding to T cell receptor (TCR) complexes.

Trifunctional antibodies (trAb) are a specific class of bispecific antibodies recruiting and activating T cells and, in particular, accessory immune cells, such as macrophages, dendritic cells, natural killer (NK) cells, and other Fc-receptor-expressing cells, simultaneously at the targeted tumor by, e.g. their Fc component. Trifunctional bispecific antibodies are known from the prior art. Reference is made in this respect to e.g. EP 1 315 520. Trifunctional bispecific antibodies have two antigen-binding sites. Typically, these binding sites allow the antibodies to bind to tumor cells (tumor cell surface antigens) and T cells (T cell surface antigens). Simultaneously, e.g. via their Fc portion, in particular Fcγ receptor positive accessory cells are recruited, for example monocytes/macrophages, natural killer cells, dendritic cells or other Fcγ receptor expressing cells. The simultaneous activation of these different classes of effector cells results in efficient killing of the tumor cells by various mechanisms such as phagocytosis and perforin-mediated cytotoxicity. Typically, the net effect of a trifunctional antibody is linking T cells and, in particular, Fcγ receptor positive accessory cells to tumor cells, leading to the destruction of the tumor cells.

In contrast to conventional ("ordinary") antibodies exhibiting just one single specificity, bispecific antibodies are able to bind to two different epitopes, for example one epitope on a target cell, e.g. a tumor cell, and one epitope on a T-cell, thereby "redirecting" the T cell to the target cell, resulting in T-cell mediated cell killing. Trifunctional antibodies evoke the removal of tumor cells in particular by means of (i) antibody-dependent cell-mediated cytotoxicity, (ii) T-cell mediated cell killing, and (iii) induction of anti-tumor immunity. In contrast, only the first mode of action is actually executed by conventional (monoclonal and monospecific) antibodies. Moreover, in contrast to conventional antibodies, bispecific, and in particular trifunctional, antibodies have a higher cytotoxic potential and they even bind to antigens, which are expressed relatively weakly. Thus, bispecific, and in particular trifunctional, antibodies are at an equivalent dose more potent (more than 1000-fold) in eliminating tumor cells compared to conventional antibodies.

A well-known example of trifunctional bispecific antibodies is catumaxomab (Removab®) (anti-EpCAM x anti-CD3). It is the only bispecific antibody, which was approved by the Authorities in April 2009 for treatment of malignant ascites (MA) in patients with EpCAM-positive carcinomas where standard therapy is not available or no longer feasible.

Further examples of trifunctional bispecific antibodies include (i) FBTA05 (also called "Lymphomun"), a trifunctional anti-CD3 x anti-CD20 antibody, which was shown to induce prompt antitumor responses in extensively pretreated, p53-mutated alemtuzumab and rituximab refractory patients, indicating its therapeutic potential (Buhmann et al. (2009) Bone Marrow Transplant, 43: 383-397), (ii) Ertumaxomab, a trifunctional anti-CD3 x anti-HER2 antibody, which was shown to induce antitumor responses in patients with metastatic breast cancer expressing HER2/neu (Kiewe et al. (2006) Clin Cancer Res, 12: 3085-3091) and (iii) TRBs02/TRBs07, two trifunctional antibodies specific for human melanoma (Ruf et al. (2004) Int J Cancer, 108: 725-732).

According to the differences in the modes of action, the established treatment protocols for bispecific, and in particular trifunctional, antibodies usually differ considerably from that for conventional (monoclonal and monospecific) antibodies. For example, catumaxomab was administered according to a regimen of four intraperitoneal infusions on days 0, 3, 7, and 10 at doses of 10, 20, 50, and 150 µg, respectively (cf. Heiss et al. (2010) Int J Cancer, 127: 2209-2221 and "Assessment Report for Removab", European Medicines Agency, Doc.Ref.: EMEA/CHMP/100434/2009). FBTA05/Lymphomun was administered as e.g. an i.v. infusion with escalating doses, starting with 10 µg on day 1, increasing, e.g. doubling, the doses on the following days, with final doses of up to 2000 µg (Buhmann et al. (2009) Bone Marrow Transplant, 43: 383-397: Table 2). Ertumaxomab was also administered as an i.v. infusion with escalating doses, starting with 10 µg on day 1, followed by 20, 50, 100, 150 or 200 µg, respectively, on each, day 7±1 and day 13±1 (Kiewe et al. (2006) Clin Cancer Res, 12: 3085-3091). Thus, for bispecific, in particular trifunctional, antibodies in clinical trials, the treatment protocol followed an escalating dosing scheme, whereby initial doses never exceeded 10 µg. It is to be noted that the initial dose for bispecific antibodies, and in particular for trifunctional antibodies, is a critical parameter, as bispecific, in particular trifunctional, antibodies activate T cells, trigger the release of proinflammatory cytokines and up-regulate co-stimulatory factors, e.g. CD28.

Cross-linking of the target cell and a T cell by bispecific antibodies, and in particular crosslinking the three cell types by trifunctional antibodies, leads to the release of cytokines, for example to the release of proinflammatory cytokines, e.g. IL-6, TNF-α or IL-8, resulting in adverse effects like fever, nausea, vomiting and chills. Such adverse effects were observed when administering bispecific, in particular trifunctional, antibodies according to the established treatment protocols described above (cf. for catumaxomab: Heiss et al. (2010) Int J Cancer, 127: 2209-2221 and "Assessment Report for Removab", European Medicines Agency, Doc.Ref.: EMEA/CHMP/100434/2009; for FBTA05/Lymphomun: Buhmann et al. (2009) Bone Marrow Transplant, 43: 383-397; for ertumaxomab: Kiewe et al. (2006) Clin Cancer Res, 12: 3085-3091; and for TRBs02/TRBs07 (in vitro only): Ruf et al. (2004) Int J Cancer, 108: 725-732). Specifically, Buhmann et al. reported that - upon application of the trifunctional bispecific antibody FBTA05 (anti-CD20 x anti-CD3) - high levels of proinflammatory cytokines are released systemically (Buhmann et al. (2009) Bone Marrow Transplant, 43: 383-397). Thus, despite the unprecedented anti-tumor activity of bispecific, in particular trifunctional, antibodies, their immunological mode of action triggers unwanted "side" effects, i.e. in the induction of unwanted inflammatory reactions known e.g. as "first dose cytokine response or syndrome".

More specifically, catumaxomab therapy is often associated with nausea, vomiting, stomach ache and inflammatory reactions, such as fever. Under such circumstances, patients need to be subjected to a concomitant treatment or premedication with e.g. analgesics, antipyretics, and/or nonsteroidal antiphlogistics (cf. "Assessment Report for Removab", European Medicines Agency, Doc.Ref.: EMEA/CHMP/100434/2009). Similarly, inflammatory reactions, such as fever, nausea and chilling, were observed for other bispecific, in particular trifunctional, antibodies as well (Buhmann et al. (2009) Bone Marrow Transplant, 43: 383-397, Kiewe et al. (2006) Clin Cancer Res, 12: 3085-3091).

Other reports further corroborate strong inflammatory reactions upon administration of T-cell redirecting bispecific, in particular trifunctional, antibodies (Stemmler et al. (2005) Anticancer Res, 25: 3047-3054; Sebastian et al. (2007) Cancer Immunol Immunother, 56: 1637). Moreover, the induction of clinical symptoms, such as fever or chills may even lead to a syndrome defined as SIRS (Systemic Inflammatory Response Syndrome) reaction under extraordinary circumstances (Stemmler et al. (2005) Anticancer Res, 25: 3047-3054). All of these clinical reactions are induced and accompanied by the release of proinflammatory cytokines; i.e. cytokines which promote systemic inflammation; e.g. IL-6, IL-8, IFN-gamma or TNF-α. In addition, it was observed that bispecific, in particular trifunctional, antibodies trigger not only the release of proinflammatory cytokines, e.g. IL-6 or IL-8, but also - simultaneously - the release of inhibitory cytokines, e.g. IL-10. IL-10 (Interleukin-10), also known as human cytokine synthesis inhibitory factor (CSIF), is an anti-inflammatory cytokine and is capable of inhibiting synthesis of proinflammatory cytokines such as IFN-γ, IL-2, IL-3, TNF-α and GM-CSF made by cells such as macrophages and regulatory T-cells. Thus, factors, such as IL-10, are released by the immune system for establishing a negative feedback loop to control inflammation. In humans, IL-10 is produced by activated CD8(+) peripheral blood T-cells, by T-helper CD4(+) T-cells after antigen-specific activation, by B-cell lymphomas, and by monocytes following cell activation by bacterial lipopolysaccharides and mast cells. Due to its macrophage inhibitory function, IL-10 is a prototypic example of a negative feedback regulator in inflammation; i.e. promoting the negative feedback regulatory function of the inflammatory arm of the immune system, thereby stabilising the immune system. Activated macrophages secret proinflammatory cytokines and upregulate co-stimulatory factors that enhance T-cell activation and cell-mediated immunity. In short, IL-10 acts on activated macrophages to weaken or to even terminate such stimulatory responses and to allow the immune system to return to its resting state.

As bispecific, in particular trifunctional, antibodies have the potential to not only kill tumor target cells directly, but to also induce antitumor immunity (and thereby induce an indirect anti-tumor response), it would be desired to reduce or ideally suppress the release of counteracting cytokines, such as IL-10 and, thereby, to prevent the proinflammatory response from being blocked by such negative feedback regulators.

Taken together, despite their above described advantages, bispecific, in particular trifunctional, antibodies evoke by their modes of action undesireable side effects, e.g. triggering the release of (i) proinflammatory cytokines, e.g. IL-6 or IL-8, resulting in adverse effects like fever, nausea, vomiting and chills, and the release of (ii) inhibitory cytokines, such as IL-10, which counteract the desired induction of anti-tumor immunization. No progress has been made in the art yet to suppress the release of proinflammatory cytokines, e.g. IL-6 or IL-8, or of inhibitory cytokines, such as IL-10, following bispecific, in particular trifunctional, antibody administration.

Thus, there is an unmet need for modifying the cytokine release profile of T-cell redirecting trifunctional multispecific; e.g. bispecific, antibodies upon their administration to humans or animals. Thereby it is envisaged to not only reduce the "first dose cytokine response", but to reduce, in parallel, an immunological counter-reaction emerging whenever strong or exceeding inflammation is dampened by negative feedback mechanisms.

These issues are addressed and solved by the embodiments characterized in the claims.

### SUMMARY OF THE INVENTION

The inventors of the present invention have surprisingly observed that the treatment protocol itself may have an impact on the cytokine release profile induced by the administration of bispecific, T-cell redirecting antibodies, in particular bispecific, trifunctional T-cell redirecting antibodies. More specifically, it was observed by the inventors that the subcutaneous route of administration directs the immune system to a significantly reduced release of proinflammatory cytokines compared to other administration routes (e.g. intravenous or intraperitoneal administration) thereby avoiding undesired side effects like vomiting and, importantly, simultaneously substantially suppresses the release of inhibitory cytokines, such as IL-10.

Thus, it was in particular the object of the present invention to administer bispecific, in particular trifunctional, antibodies in such a manner that the cytokine profile is modified as described above. Namely, in particular the release of proinflammatory cytokines and/or of inhibitory cytokines is to be decreased. Thereby, (i) side effects of administration of bispecific, in particular trifunctional, antibodies due to release of proinflammatory cytokines are reduced and/or (ii) anti-tumor immunization by bispecific, in particular trifunctional, antibodies is increased since inhibition of anti-tumor immunization by inhibitory cytokines, e.g. IL-10, is decreased. Accordingly, the present invention relates to a bispecific, T-cell redirecting antibody comprising a specificity against a tumor-associated antigen and an Fc region for use in the treatment of cancer wherein said antibody is administered via the subcutaneous route. Based thereon, reducing the release of proinflammatory cytokines and simultaneously decreasing, preferably substantially suppressing, the release of inhibitory cytokines occurring in a mammal e.g. after intravenous or intraperitoneal administration of a T-cell redirecting bispecific, in particular trifunctional, antibody can be achieved by administering the bispecific, in particular trifunctional, antibody subcutaneously. Preferably, an appropriate dosing (dosing regimen) is applied for administering the bispecific, in particular trifunctional, antibodies subcutaneously.

Preferably, the bispecific, in particular trifunctional, antibodies employed according to the present invention are capable of reactivating tumor-specific T cells being in the anergic state. Furthermore, the trifunctional antibodies are preferably capable of inducing tumor-reactive complement-binding antibodies and therefore induce a humoral immune response.

The bispecific, in particular trifunctional, antibody for use in the treatment of cancer according to the present invention comprises a specificity against a tumor-associated antigen, to recruit tumor cells. Preferably, the tumor-associated antigen (or an epitope thereon, respectively) to be recognized by the bispecific, in particular trifunctional, antibody is selected from the group consisting of EpCAM, HER2/neu, HER3/neu, G250, CEA, MAGE, proteoglycans, VEGF, EGFR, .alpha.V.beta.3-integrin, HLA, HLA-DR, ASC, CD1, CD2, CD4, CD6, CD7, CD8, CD11, CD13, CD14, CD19, CD20, CD21, CD22, CD23, CD24, CD30, CD33, CD37, CD40, CD41, CD47, CD52, c-erb-2, CALLA, MHCII, CD44v3, CD44v6, p97, ganglioside GM1, GM2, GM3, GD1a, GD1b, GD2, GD3, GT1b, GT3, GQ1, NY-ESO-1, NFX2, SSX2, SSX4 Trp2, gp100, tyrosinase, Muc-1, telomerase, survivin, G250, p53, CA125 MUC, Wue antigen, Lewis Y antigen, HSP-27, HSP-70, HSP-72, HSP-90, Pgp, MCSP, EpHA2 and cell surface targets GC182, GT468 or GT512. It is also preferred that the tumor-associated antigen (or an epitope thereon, respectively) to be recognized by the bispecific, in particular trifunctional, antibody is selected from the group consisting of EpCAM, HER2/neu, HER3/neu, G250, CEA, MAGE, proteoglycans, VEGF, EGFR, .alpha.V.beta.3-integrin, HLA, HLA-DR, ASC, CD1, CD2, CD4, CD6, CD7, CD8, CD11, CD13, CD14, CD19, CD20, CD21, CD22, CD23, CD24, CD30, CD33, CD37, CD40, CD41, CD47, CD52, c-erb-2, CALLA, MHCII, CD44v3, CD44v6, p97, ganglioside GM1, GM2, GM3, GD1a, GD1b, GD2, GD3, GT1b, GT3, GQ1, NY-ESO-1, NFX2, SSX2, SSX4 Trp2, gp100, tyrosinase, Muc-1, telomerase, survivin, G250, p53, CA125 MUC, Wue antigen, Lewis Y antigen, HSP-27, HSP-70, HSP-72, HSP-90, Pgp, MCSP, EpHA2 and cell surface targets GC182, GT468, PD-L1, arboviral E protein epitope or GT512.

Preferably, the tumor-associated antigen (or an epitope thereon, respectively) to be recognized by the bispecific, in particular trifunctional, antibody is EpCAM. Preferably, the tumor-associated antigen (or an epitope thereon, respectively) to be recognized by the bispecific, in particular trifunctional, antibody is Her2/neu. Preferably, the tumor-associated antigen (or an epitope thereon, respectively) to be recognized by the bispecific, in particular trifunctional, antibody is GD2. Preferably, the tumor-associated antigen (or an epitope thereon, respectively) to be recognized by the bispecific, in particular trifunctional, antibody is GD3. Preferably, the tumor-associated antigen (or an epitope thereon, respectively) to be recognized by the bispecific, in particular trifunctional, antibody is CD20. Preferably, the tumor-associated antigen (or an epitope thereon, respectively) to be recognized by the bispecific, in particular trifunctional, antibody is CD19. Preferably, the tumor-associated antigen (or an epitope thereon, respectively) to be recognized by the bispecific, in particular trifunctional, antibody is CD30. Preferably, the tumor-associated antigen (or an epitope thereon, respectively) to be recognized by the bispecific, in particular trifunctional, antibody is CD33. Preferably, the tumor-associated antigen (or an epitope thereon, respectively) to be recognized by the bispecific, in particular trifunctional, antibody is PD-L1. Preferably, the tumor-associated antigen (or an epitope thereon, respectively) to be recognized by the bispecific, in particular trifunctional, antibody is EGF-R. Preferably, the tumor-associated antigen (or an epitope thereon, respectively) to be recognized by the bispecific, in particular trifunctional, antibody is an arboviral E protein epitope.

Accordingly, the bispecific, in particular trifunctional, antibody structure encompasses one specificity, in particular one paratope, which recognizes an epitope of a tumor antigen, preferably as described above, in particular selected from the above list, more preferably selected from EpCAM, Her2/neu and GD2, while its other specificity, in particular its other paratope, recognizes an epitope of a T-cell surface antigen. As used herein, "paratope" refers to an antigen-binding site of the antibody and, thus, a single "specificity" may refer to one, two, three or more identical paratopes in a single antibody. For example, a single native IgG antibody is monospecific, but has two identical paratopes. Accordingly, a bispecific antibody comprises at least two paratopes.

For recruiting T cells and tumor cells by the bispecific, in particular trifunctional, antibody to be used according to the invention, the bispecific, in particular trifunctional, antibody to be used by the present invention therefore comprises another specificity, in particular another paratope, which recognizes an epitope of a T cell surface receptor, preferably selected from the group consisting of CD3, CD2, CD4, CD5, CD6, CD8, CD28, CD40L and/or CD44, preferably CD3.

Therefore, the bispecific, in particular trifunctional, antibody for use in the treatment of cancer according to the present invention preferably binds by its first specificity, e.g. by its first paratope, to an epitope of the T-cell surface antigen, preferably CD3, and, by its second specificity, e.g. by its second paratope, to a tumor-associated antigen preferably selected from the group consisting of the tumor antigens EpCAM, HER2/neu, CD20 or the ganglioside GM1, GM2, GM3, GD1a, GD1b, GD2, GD3, GT1b, GT3 or GQ1; more preferably, said bispecific, in particular trifunctional, antibody binds to CD3 by its first specificity, in particular by its first paratope, and, by its second specificity, in particular by its second paratope, to the tumor-associated antigen GD2; or alternatively preferably, said bispecific, in particular trifunctional, antibody binds to CD3 by its first specificity, in particular by its first paratope, and, by its second specificity, in particular by its second paratope, to the tumor antigen CD20; or alternatively preferably, said bispecific, in particular trifunctional, antibody binds to CD3 by its first specificity, in particular by its first paratope, and, by its second specificity, in particular by its second paratope, to tumor antigen EpCAM.

Preferably, a bispecific, in particular trifunctional, antibody to be used according to the invention is selected such that it exhibits, on the one hand, specificity to a T-cell surface antigen, e.g. CD3, and, on the other hand, specificity to a tumor-associated antigen, e.g. GD2 (anti-CD3 X anti-GD2) or CD20 (anti-CD3 X anti-CD20) or EpCAM (anti-CD3 x anti-EpCAM), or HER2 (anti-CD3 x anti-HER2) most preferably catumaxomab (anti-CD3 x anti-EpCAM), FBTA05/Lymphomun (anti-CD3 X anti-CD20), Ektomun (anti-CD3 X anti-GD2)or ertumaxomab (anti-CD3 x anti-HER2).

Preferably, a bispecific, in particular trifunctional, antibody to be used according to the present invention comprises one specificity, preferably one paratope, against CD3 and one specificity, preferably one paratope, against EpCAM (anti-CD3 x anti-EpCAM). Preferably, a bispecific, in particular trifunctional, antibody to be used according to the present invention comprises one specificity, preferably one paratope, against CD3 and one specificity, preferably one paratope, against Her2/neu (anti-CD3 x anti-Her2/neu). Preferably, a bispecific, in particular trifunctional, antibody to be used according to the present invention comprises one specificity, preferably one paratope, against CD3 and one specificity, preferably one paratope, against GD2 (anti-CD3 x anti-GD2). Preferably, a bispecific, in particular trifunctional, antibody to be used according to the present invention comprises one specificity, preferably one paratope, against CD3 and one specificity, preferably one paratope, against GD3 (anti-CD3 x anti-GD3). Preferably, a bispecific, in particular trifunctional, antibody to be used according to the present invention comprises one specificity, preferably one paratope, against CD3 and one specificity, preferably one paratope, against CD20 (anti-CD3 x anti-CD20). Preferably, a bispecific, in particular trifunctional, antibody to be used according to the present invention comprises one specificity, preferably one paratope, against CD3 and one specificity, preferably one paratope, against CD19 (anti-CD3 x anti-CD19). Preferably, a bispecific, in particular trifunctional, antibody to be used according to the present invention comprises one specificity, preferably one paratope, against CD3 and one specificity, preferably one paratope, against CD30 (anti-CD3 x anti-CD30). Preferably, a bispecific, in particular trifunctional, antibody to be used according to the present invention comprises one specificity, preferably one paratope, against CD3 and one specificity, preferably one paratope, against CD33 (anti-CD3 x anti-CD33). Preferably, a bispecific, in particular trifunctional, antibody to be used according to the present invention comprises one specificity, preferably one paratope, against CD3 and one specificity, preferably one paratope, against PD-L1 (anti-CD3 x anti-PD-L1). Preferably, a bispecific, in particular trifunctional, antibody to be used according to the present invention comprises one specificity, preferably one paratope, against CD3 and one specificity, preferably one paratope, against EGF-R (anti-CD3 x anti-EGF-R). Preferably, a bispecific, in particular trifunctional, antibody to be used according to the present invention comprises one specificity, preferably one paratope, against CD3 and one specificity, preferably one paratope, against an arboviral E protein epitope (anti-CD3 x anti- arboviral E protein).

Trifunctional antibodies which may be employed according to the present invention exhibit three functions, in particular including their ability to bind (redirect) T-cells and to bind (target) tumor cells by the two specificities as described above, and - as a third function - trifunctional antibodies are in particular able to bind e.g. to monocytes, macrophages, dendritic cells, "natural killer" cells (NK cells) and/or activated neutrophils, in particular by their Fc receptor. Preferably, their Fc portion binds to Fc receptor-positive cells, which preferably at least express one type of an Feγ receptor, preferably an Fcγ receptor type I, IIa and/or III.

As the trifunctional bispecific antibody to be used according to the invention recruits in particular by its Fc portion immune cells expressing on its cell surface Fc receptors, in particular Fcγ receptor type I, IIa and/or III, the trifunctional bispecific antibodies in particular aggregate three distinct cell types, i.e. two types of immune cells (T cells and Fc receptor expressing immune cells) and the target tumor cells. Various modes of action of the immune system are thereby mobilized to attack the recruited target tumor cell.

Preferably, a trifunctional bispecific antibody to be used according to the invention is an IgG type antibody. Preferably, a trifunctional bispecific antibody to be used according to the invention is a heterologous intact rat/mouse antibody, whereby an antibody with a subclass combination of mouse IgG2a and rat IgG2b is preferred. A heterologous intact rat/mouse antibody with a heavy chain composed of murine IgG2a and rat IgG2b subclasses, each with their respective light chains, is particularly preferred.

In general, a trifunctional bispecific antibody to be used according to the invention exhibits preferably one of the following isotype combinations in its Fc-region: rat-IgG2b/mouse-IgG2a, rat-IgG2b/mouse-IgG2b, rat-IgG2b/human-IgG1, or mouse-[VH-CH1,VL-CL]-human-IlgG1/rat-[VH-CH1,VL-CL]-human-IgG1-[hinge]-human IgG3*-[CH2-CH3], wherein * = caucasian allotypes G3m(b+g) = no binding to protein A.

By the subcutaneous route of administration, the antibody for use according to the present invention decreases, preferably substantially avoids or suppresses, the release of inhibitory cytokines, which may be selected from IL-10, IL-35 and TGF-β, whereas the release of proinflammatory cytokines, which may be selected from IL-6, IL-8, IFN-γ and TNF-α, is reduced. Subcutaneous administration essentially means administration of the bispecific, in particular trifunctional, antibody to subcutaneous tissue, in particular to the fat layer beneath the dermis. Subcutaneous administration may be enabled by needle or needle-free administration or by infusion including the use of infusion pumps.

The cancer disease to be treated by the bispecific antibody, in particular trifunctional, according to the present invention may be selected from the group consisting of solid or non-solid tumors, preferably selected from the group consisting of ectodermal, in particular neuroectodermal, neurogenic, mesenchymal or epithelial origin and metastases thereof, colon carcinomas, melanomas, squamous cell carcinomas, renal carcinomas, lymphomas, preferably Hodgkin lymphoma or non-Hodgkin lymphoma, e.g. diffuse large B-cell lymphoma (DLBCL), Ewing's sarcoma, lipsarcome, fibrosarcoma, leiomyosarcoma, meloma, thymoma, acute myeloid leukaemia (AML), acute lymphoid leukaemia (ALL), chronic myeloid leukaemia (CML), chronic lymphocytic leukaemia (CLL), gastrointestinal tumors, pulmonary carcinomas, gliomas, thyroid tumours, mammary carcinomas, prostate tumours, hepatomas, various virus-induced tumours such as, for example, papilloma virus-induced carcinomas (e.g. cervical carcinoma), adenocarcinomas, herpes virus-induced tumours (e.g. Burkitt's lymphoma, EBV-induced B-cell lymphoma), hepatitis B-induced tumours (hepatocell carcinoma), HTLV-1 and HTLV-2 induced lymphomas, acoustic neuromas/neurinomas, cervical cancer, lung cancer, pharyngeal cancer, anal carcinomas, neuroblastomas, gliomas, glioblastomas, lymphomas, rectal carcinomas, astrocytomas, brain tumours, stomach cancer, retinoblastomas, basaliomas, brain metastases, medulloblastomas, vaginal cancer, pancreatic carcinoma, testicular cancer, melanomas, thyroidal carcinomas, bladder cancer, Hodgkin's syndrome, meningiomas, Schneeberger disease, bronchial carcinomas, hypophysis tumour, Mycosis fungoides, oesophageal cancer, breast cancer, cardinoids, neurinomas, spinaliomas, laryngeal cancer, renal cancer, thymomas, corpus carcinomas, bone cancer, osteosarcomas, urethral cancer, CUP syndrome, head/neck tumours, oligodendrogliomas, vulval cancer, intestinal cancer, colon carcinomas, oesophageal carcinomas, tumors of the small intestine, craniopharyngeomas, ovarian carcinomas, gastric carcinoma, colonic carcinoma, soft tissue tumours/sarcomas, ovarian cancer, liver cancer, pancreatic carcinomas, cervical carcinomas, endometrial carcinomas, liver metastases, hepatocellular carcinoma, penile cancer, tongue cancer, gall bladder cancer, leukemia, plasmocytomas, uterine cancer, lid tumour, prostate cancer, malignant melanoma and blastomas. Particularly, solid tumours of ectodermal, in particular neuroectodermal, neurogenic, mesenchymal or epithelial origin and metastases thereof or non-solid tumors of the lymphatic or myeloid system may be addressed by the use according to the present invention.

The bispecific, in particular trifunctional, antibody to be used according to the invention may preferably serve for the treatment of solid or non-solid cancer diseases selected from the group consisting of gastric carcinoma, adenocarcinoma, melanoma, in particular malignant melanoma, glioma, colonic carcinoma, pancreatic carcinoma, ovarian carcinoma, uterine cancer, breast cancer, in particular metastatic breast cancer, hepatocellular carcinoma, bronchial carcinoma, leukemias, preferably acute or chronic leukemias of myeloid or lymphoid origin, lymphomas, preferably Hodgkin-lymphoma, non-Hodgkin lymphoma, e.g. diffuse large B-cell lymphoma (DLBCL), Ewing's sarcoma, liposarcome, fibrosarcoma, leiomyosarcoma, myeloma, thymoma and other soft tissue sarcomas and blastomas. More specifically, blastomas may be selected from the group consisting of hepatoblastoma, medulloblastoma, nephroblastoma, neuroblastoma, pancreatoblastoma, pleuropulmonary blastoma, retinoblastoma and glioblastoma.

Preferably, the bispecific, in particular trifunctional, antibody to be used according to the present invention is used for the treatment of Hodgkin-lymphoma or non-Hodgkin lymphoma, e.g. diffuse large B-cell lymphoma (DLBCL).

The mammal to be treated is human or non-human animal. Amongst humans, the inventive treatment protocol by administering bispecific, in particular trifunctional, antibodies subcutaneously may be particularly useful for the treatment of any group of cancer patients. In particular, young (less than 15 year old) or elderly (more than 60 year old) cancer patients, in particular young and elderly patients suffering from non-solid tumors, e.g. lymphomas or leukemia, may be treated according to the present invention. Cancer patients, irrespective of their age, who are not under immunosuppressive treatment and, in particular, who may have not received an allogenic stem cell transplantation, may particularly benefit from the use of the bispecific, in particular trifunctional, antibody according to the invention.

The invention is typically characterized by a treatment protocol defining the administration of various doses (starting with an initial dose), which is - over time - increased by administering higher doses until a maximum dose is reached. A dosing regimen starting with an initial dose and ending with a maximum dose is herewith defined as one treatment cycle. Typically, the patient to be treated with the bispecific, in particular trifunctional, antibody according to the present invention receives several treatment cycles (which are typically interrupted by periods without bispecific antibody, in particular trifunctional, administration). The final dose of a treatment cycle typically reflects the highest amount of antibody to be administered within one treatment cycle; i.e. the maximum dose. In a preferred embodiment, a (single) dose of the bispecific, in particular trifunctional, antibody is subcutaneously administered two or three times a week with a pause of 1-3 days between the injections. In another preferred embodiment, a (single) dose of the bispecific, in particular trifunctional, antibody is subcutaneously administered daily. Daily administration is preferably for 2, 3, 4, 5, 6, 7, 8, 9, 10, 11, 12, 13, 14, 15, 16, 17, 18, 19, 20, 21, 22, 23, 24, 25, 26, 27 or 28 consecutive days.

A single dose of the bispecific, in particular trifunctional, antibody to be administered will preferably include 10 to 3000 µg of said antibody, which is preferably administered daily. Typically, the bispecific, in particular trifunctional, antibody according to the present invention is administered at an initial dose of 10 µg to 200 µg or more than 40 µg; preferably from 10 µg to 100 µg, more preferably from 20 µg to 80 µg, even more preferably from 40 µg to 60 µg, e.g. 50 µg subcutaneously. However, for example the trifunctional antibody Ektomun may be administered at an initial dose of up to 200 µg, preferably 100 µg to 200 µg, more preferably 150 µg to 200 µg, for example 200µg. The maximum dose (within a treatment cycle) is typically selected from a range of 500 µg to 3000 µg, preferably from 500 µg to 1500 µg, e.g. 800 µg or more, e.g. 800 µg - 10000 µg, 1500 µg or more, e.g. 1500 µg - 7500 µg, or 2000 µg or more, e.g. 2000 µg - 5000 µg, subcutaneously. Accordingly, the maximum dose to be administered typically includes 800 µg or more, preferably 800 µg - 10000 µg, of said antibody.

It is also preferred that a single dose of the bispecific, in particular trifunctional, antibody to be administered includes 10 to 10000 µg of said antibody, which is preferably administered daily. Preferably, the bispecific, in particular trifunctional, antibody according to the present invention is administered at an initial dose of 10 µg to 500 µg or more than 40 µg, e.g. 50 µg, 100 µg or 200 µg; preferably from 10 µg to 100 µg, more preferably from 20 µg to 80 µg, even more preferably from 40 µg to 60 µg, e.g. 50 µg subcutaneously. It is also preferred that the bispecific, in particular trifunctional, antibody according to the present invention is administered at an initial dose of 50 µg to 150 µg, more preferably from 70 µg to 130 µg, even more preferably from 90 µg to 110 µg, e.g. 100 µg subcutaneously. However, for example the trifunctional antibody Ektomun may be administered at an initial dose of up to 1000 µg, preferably 200 µg to 400 µg, more preferably 300 µg to 400 µg, for example 400µg. The maximum dose (within a treatment cycle) is typically selected from a range of 100 µg to 10000 µg, preferably from 500 µg to 1500 µg, e.g. 800 µg or more, 1500 µg or more or 2000 µg or more, preferably 800 µg - 10000 µg, more preferably, 1500 µg - 7500 µg, even more preferably 2000 µg - 5000 µg, subcutaneously. Accordingly, the maximum dose to be administered typically includes 800 µg or more, preferably 800 µg - 10000 µg, of said antibody.

Such a dosage regimen allows to substantially suppress the release of inhibitory cytokines, which may be reflected by IL-10, IL-35 and TGF-β and to reduce the release of proinflammatory cytokines, which may be reflected by IL-6, IL-8, INF-γ and TNF-α. The initial dose is increased, e.g. stepwise, in the course of the treatment cycle to finally arrive at a maximum dose. Correspondingly, the bispecific, in particular trifunctional, antibody as defined by the present invention is also used for the preparation of a pharmaceutical composition for the treatment of an individual diagnosed with cancer, wherein said pharmaceutical composition is preferably administered subcutaneously and wherein preferably said pharmaceutical composition is administered by such a volume that it corresponds to an intial dose of the bispecific, in particular trifunctional, antibody (dissolved in solution) from 10 µg to 200 µg, preferably from 30 µg to 150 µg, e.g. 50 µg or 100 µg and/or a maximum dose of 500 to 3000 µg, e.g. 800 µg, 1000 µg or 2000 µg. It is also preferred that the bispecific, in particular trifunctional, antibody as defined by the present invention is used for the preparation of a pharmaceutical composition for the treatment of an individual diagnosed with cancer, wherein said pharmaceutical composition is preferably administered subcutaneously and wherein preferably said pharmaceutical composition is administered by such a volume that it corresponds to an intial dose of the bispecific, in particular trifunctional, antibody (dissolved in solution) from 10 µg to 500 µg, preferably from 30 µg to 150 µg, e.g. 50 µg or 100 µg and/or a maximum dose of 100 to 10000 µg, e.g. 800 µg, 1000 µg or 2000 µg.

The present invention relates more specifically to a treatment characterized by a dosage regimen for use in the treatment of cancer, wherein a bispecific, in particular trifunctional, antibody is administered subcutaneously to a patient diagnosed with at least one type of cancer comprising (a) subcutaneously administering an initial dose of said antibody; and consecutively (b) subcutaneously administering at least one repeating dose of said antibody; wherein said at least one repeating dose is either unaltered interms of the amount of antibody administered by the previous dose or, preferably, exceeds the amount of the previous dose by a factor of from 1.5 to 5, more preferably from 1.5 to 3, and even more preferably from 1.5 to 2.5.

Preferably, administration of the bispecific, in particular trifunctional, antibody is repeated within a treatment cycle by individual doses every 1 to 10 days, more preferably every 2 to 4 days, even more preferably every 3 days. That dose, which is preferably administered every 1 to 10 days, more preferably every 2 to 4 days, even more preferably every 3 days, is preferably the "repeating dose". Each single dose is typically administered subcutaneously within 30 minutes, preferably within 10 minutes, more preferably within 5 minutes, preferably by a subcutaneous (bolus) injection or infusion, in particular subcutaneous infusion, preferably distributed to different injection sites, more preferably 2-8 injection sites, even more preferably 4 injection sites. Thus, the initial dose and/or at least one of the repeating dose(s) are preferably administered subcutaneously within 30 minutes, preferably within 10 minutes, more preferably within 5 minutes, preferably by a subcutaneous (bolus) injection or infusion, in particular subcutaneous infusion, preferably distributed to different injection sites, more preferably 2-8 injection sites, even more preferably 4 injection sites. For example, each single dose is administered subcutaneously for 1 to 30 minutes, preferably for 1 to 10 minutes, more preferably 1 to 5 minutes, preferably by a subcutaneous (bolus) injection or subcutaneous infusion, preferably by distribution to 4 different injection sites.

The bispecific, in particular trifunctional, antibody is typically provided as a pharmaceutical composition, e.g. as a lyophilised powder or, preferably, as solution, preferably as aqueous solution, more preferably as aqueous buffered solution. If the antibodies are provided as a lyophilized powder, that lyophilized antibody powder may be typically dissolved in an appropriate solvent system, preferably in an aqueous solvent system, e.g. an isotonic saline solution or PBS. Thus, the pharmaceutical composition of the invention encompasses (such) a liquid composition containing the bispecific, in particular trifunctional, antibody either in a concentrated form (to be diluted prior to its use) or as a ready-to-use composition, e.g. prefilled in a syringe.

Further described herein is a kit comprising the pharmaceutical composition as defined herein, and, optionally, a liquid, e.g. an aqueous solvent, suitable for reconstitution of the lyophilised powder. Preferably, said liquid suitable for reconstitution of the lyophilised powder may be isotonic saline or water for injection, optionally buffered.

Finally, the present invention refers to a combination therapy for use in the treatment of a cancer disease allowing the subcutaneous administration of the bispecific, in particular trifunctional, antibody to be used according to the invention to be combined with another anti-cancer treatment, an anti-cancer drug or an anti-cancer monoclonal monospecific antibody, which may be administered by any suitable route, e.g. orally, intravenously or subcutaneously, preferably by an administration being separate from the administration of the bispecific, in particular trifunctional, antibody.

### FIGURES

The figures show the following:
- Figure 1: shows the pharmakokinetik profile of the trifunctional antibody FBTA05/Lymphomun (anti-CD20 x anti-CD3) after six subcutaneous applications to a lymphoma patient.
- Figure 2: shows the release of proinflammatory cytokines such as IL-6, IL-8, and TNF-α or counter-inflammatory cytokines like IL-10. Proinflammatory cytokines could be detected in low amounts whereas inhibitory cytokines such as IL-10 were almost not detectable.
- Figure 3: demonstrates the CD19+ B-cell count during subcutaneous administration of FBTA05.
- Figure 4: shows the CD8+ /CD3+ T-cell count during subcutaneous administration of FBTA05.
- Figure 5: shows the CD4+ /CD3+ helper T-cell count during subcutaneous administration of FBTA05.
- Figure 6: shows the NK cell (CD16+, CD56+, CD3-) count during subcutaneous administration of FBTA05.
- Figure 7: shows for Example 3 the skin redness at the four sites of injection immediately after (a, c) as well as 20 h after (b, d) subcutaneous administration of 100 µg antibody (a, b) and of 2000 µg antibody (c, d).
- Figure 8: shows for Example 3 the pharmacokinetics profile of the trifunctional antibody Lymphomun after subcutaneous administration. The antibody was administered at the indicated doses and timepoints (arrows).
- Figure 9: shows for Example 3 the measurement of cytokines (Luminex multiplex technology) in the blood of the lymphoma patient. Proinflammatory cytokine IL-6 showed highest values. In addition, IL-8, TNF-α and IL-10 were detectable.
- Figure 10: shows for Example 3 the course of the CRP-level in the blood of the patient. CRP-values were determined by Clinical Chemistry Großhadern, Munich/Germany using the "high sensitive" method (hs-CRP). Normal values are below 0.5 mg/dl.
- Figure 11: shows for Example 3 the course of the LDH-level (LDH, 37°C) in the blood of the patient. LDH-values were determined by Clinical Chemistry Großhadem, Munich/Germany. LDH normal range is ≤ 250 U/I.
- Figure 12: shows for Example 3 the cutaneous lymphoma manifestation in the region of the right side of the face before (a) as well as following (b; the complete regression) the treatment with Lymphomun. In the region of the right lower leg a considerable regression is shown with restriction against the surrounding healthy tissue and concomitant regression of tumor pain following antibody treatment (d) in comparison to the initial findings (c).
- Figure 13: shows for Example 3 the depletion of CD19+ B-cells in the blood of the patient during the course of the subcutaneous antibody therapy.
- Figure 14: shows for Example 3 the course of the thrombocyte value during and after the antibody therapy. Values were determined by Clinical Chemistry Großhadern, Munich/Germany. The normal range is between 146 and 328 G/J.
- Figure 15: shows for Example 3 the course of the number of CD3+/CD45+ T-cells (A), CD8+ T-cells (B), CD4+ T-cells (C) and CD16+/CD56+ NK cells (D) in the blood of the patient. The measurement was performed using the multitest IMK kit (Becton Dickinson). Arrows indicate injection of the antibody.

### DETAILED DESCRIPTION OF THE INVENTION

The present invention relates to a bispecific, T-cell redirecting antibody comprising a specificity against a tumor-associated antigen and an Fc region for use in the treatment of cancer, wherein said antibody is administered via the subcutaneous route. Based thereon, the present invention allows to reduce the release of proinflammatory cytokines and, simultaneously, to substantially suppress the release of inhibitory cytokines occurring in a mammal after intravenous or intraperitoneal administration of a T-cell redirecting bispecific, in particular trifunctional, antibody.

Additionally, the present invention relates to a pharmaceutical composition for use in the treatment of an individual diagnosed with cancer, whereby the pharmaceutical composition contains at least one type of a bispecific, in particular trifunctional, antibody as defined herein.

As used herein, the term "bispecific antibody" refers to a protein, which has the ability to bind to two different epitopes on the same or different antigens. A bispecific antibody therefore typically has two different (types of) paratopes, i.e. antigen-binding sites. In particular, a bispecific antibody may comprise two or more paratopes, wherein some paratopes may be identical so that all paratopes of the antibody belong to only two different types of paratopes and, hence, the antibody has two specificities. For example, the bispecific, in particular trifunctional, antibody according to the present invention may comprise four paratopes, wherein each two paratopes are identical (i.e. have the same specificity) and, thus, the antibody is bispecific (two identical paratopes for each of the two specificities). As used herein, "one specificity" in particular refers to one or more paratopes exhibiting the same specificity (which typically means that such one or more paratopes are identical) and, thus, "two specificities" may be realized by two, three, four five, six or more paratopes as long as they refer to only two specificities. Preferably a single antibody comprises one single paratope for each of the two specificities, i.e. the antibody comprises in total two paratopes. Preferably the antibody comprises two (identical) paratopes for each of the two specificities, i.e. the antibody comprises in total four paratopes. Preferably the antibody comprises three (identical) paratopes for each of the two specificities, i.e. the antibody comprises in total six paratopes. More preferably, the antibody comprises one single paratope for each of the two specificities, i.e. the antibody comprises in total two paratopes. More preferably, the antibody comprises two (identical) paratopes for each of the two specificities, i.e. the antibody comprises in total four paratopes. Most preferably, the antibody comprises one single paratope for each of the two specificities, i.e. the antibody comprises in total two paratopes.

A bispecific antibody may be an antibody, an antibody-derived protein, or, for example, a recombinant protein exhibiting antigen binding sites. In particular, bispecific antibodies (BiAbs) encompass "whole" antibodies, such as whole IgG- or IgG-like molecules, and small recombinant formats, as long as they comprise an Fc region. Examples of bispecific antibody formats include quadroma.

Preferably, the bispecific, T-cell redirecting antibody comprising a specificity against a tumor-associated antigen and an Fc region as used according to the present invention is selected from the group comprising Triomabs; hybrid hybridoma (quadroma); Multispecific anticalin platform (Pieris); Diabodies; Single chain diabodies; Duobody platform (Genmab); Dock and lock platform; Knob into hole (KIH) platform; Humanized bispecific IgG antibody (REGN1979) (Regeneron); Mab² bispecific antibodies (F-Star); DVD-Ig = dual variable domain immunoglobulin (Abbvie); kappa-lambda bodies; TBTI = tetravalent bispecific tandem Ig; and CrossMab.

Preferably, bispecific antibodies as used according to the present invention may be selected from bispecific IgG-like antibodies (BsIgG) comprising CrossMab; DAF (two-in-one); DAF (four-in-one); DutaMab; DT-IgG; Knobs-in-holes common LC; Knobs-in-holes assembly; Charge pair; Fab-arm exchange; SEEDbody; Triomab; LUZ-Y; Fcab; κλ-body; and Orthogonal Fab. These bispecific antibody formats are shown and described for example in Spiess C., Zhai Q. and Carter P.J. (2015) Molecular Immunology 67: 95-106, in particular Fig. 1 and corresponding description, e.g. p. 95 - 101.

Preferably, bispecific antibodies as used according to the present invention may be selected from IgG-appended antibodies with an additional antigen-binding moiety comprising DVD-IgG; IgG(H)-scFv; scFv-(H)IgG; IgG(L)-scFv; scFV-(L)IgG; IgG(L,H)-Fv; IgG(H)-V; V(H)-IgG; IgG(L)-V; V(L)-IgG; KIH IgG-scFab; 2scFv-IgG; IgG-2scFv; scFv4-Ig; scFv4-Ig; Zybody; and DVI-IgG (four-in-one). These bispecific antibody formats are shown and described for example in Spiess C., Zhai Q. and Carter P.J. (2015) Molecular Immunology 67: 95-106, in particular Fig. 1 and corresponding description, e.g. p. 95 - 101.

The antibody according to the present invention may preferably be based on any immunoglobulin class (e.g., IgA, IgG, IgM) and subclass (e.g. IgA1, IgA2, IgG1, IgG2, IgG3, IgG4). In the present invention an antibody having an IgG-like format is preferred (based on IgG, also referred to as "IgG type"), whereby an antibody having an IgG-like format usually comprises two heavy chains and two light chains. Examples of an antibody having an IgG-like format include a quadroma and various IgG-scFv formats (cf: Byrne H. et al. (2013) Trends Biotech, 31 (11): 621-632; Figure 2A-E), whereby a quadroma is preferred, which is preferably generated by fusion of two different hybridomas. Within the IgG class, antibodies may preferably be based on the IgG1, IgG2, IgG3 or IgG4 subclass, whereby an antibody based on IgG1 (also referred to as "IgG1 type") is preferred.

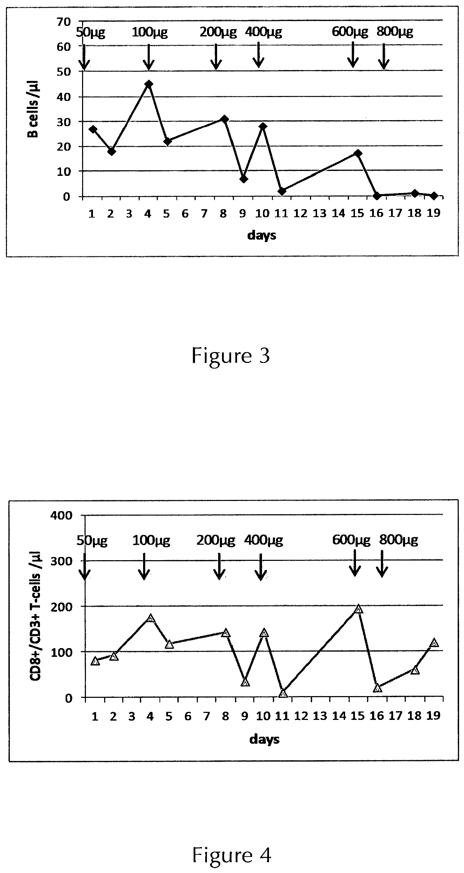

Preferred bispecific IgG-like antibody formats comprise for example hybrid hybridoma (quadroma), knobs-into-holes with common light chain, various IgG-scFv formats, various scFv-IgG formats, two-in-one IgG, dual V domain IgG, IgG-V, and V-IgG, which are shown for example in Figure 3c of Chan, A.C. and Carter, P.J. (2010) Nat Rev Immu 10: 301-316 and described in said article. Further preferred bispecific IgG-like antibody formats include for example DAF, CrossMab, IgG-dsscFv, DVD, IgG-dsFV, IgG-scFab, scFab-dsscFv and Fv2-Fc, which are shown in Fig. 1A of Weidle U.H. et al. (2013) Cancer Genomics and Proteomics 10: 1 - 18 and described in said article. Further preferred bispecific IgG-like antibody formats include DAF (two-in-one); DAF (four-in-one); DutaMab; DT-IgG; Knobs-in-holes assembly; Charge pair; Fab-arm exchange; SEEDbody; Triomab; LUZ-Y; Fcab; κλ-body; Orthogonal Fab; DVD-IgG; IgG(H)-scFv; scFv-(H)IgG; IgG(L)-scFv; scFV-(L)IgG; IgG(L,H)-Fv; IgG(H)-V; V(H)-IgG; IgG(L)-V; V(L)-IgG; KIH IgG-scFab; 2scFv-IgG; IgG-2scFv; scFv4-Ig; scFv4-Ig; Zybody; and DVI-IgG (four-in-one) as shown and described for example in Spiess C., Zhai Q. and Carter P.J. (2015) Molecular Immunology 67: 95-106, in particular Fig. 1 and corresponding description, e.g. p. 95 - 101.

In general, bispecific antibodies are produced by three main methods: (i) chemical conjugation, which involves chemical cross-linking; (ii) fusion of two different hybridoma cell lines; or (iii) genetic approaches involving recombinant DNA technology. The fusion of two different hybridomas produces a hybrid-hybridoma (or "quadroma") secreting a heterogeneous antibody population including bispecific molecules. Alternative approaches included chemical conjugation of two different mAbs and/or smaller antibody fragments. Oxidative reassociation strategies to link two different antibodies or antibody fragments were found to be inefficient due to the presence of side reactions during reoxidation of the multiple native disulfide bonds. Current methods for chemical conjugation focus on the use of homo-or hetero-bifunctional crosslinking reagents. Recombinant DNA technology has yielded the greatest range of bsAbs, through artificial manipulation of genes and represents the most diverse approach for bsAb generation (45 formats in the past two decades; cf. Byrne H. et al. (2013) Trends Biotech, 31 (11): 621-632).

In particular by use of such recombinant DNA technology, also a variety of further multispecific antibodies have emerged recently. The term "multispecific antibodies" refers to proteins having more than one paratope and the ability to bind to two or more different epitopes. Thus, the term "multispecific antibodies" comprises bispecific antibodies as defined above, but typically also protein, e.g. antibody, scaffolds, which bind in particular to three or more different epitopes, i.e. antibodies with three or more paratopes. Such multispecific proteins, in particular with three or more paratopes, are typically achieved by recombinant DNA techniques. In the context of the present invention, the antibody may in particular also have more than two specificities, and, thus, more than two paratopes, as at least two paratopes are required according to the present invention, for example one for the target cell and the other for a T cell. Accordingly, the antibody to be used according to the invention may have further paratopes, in particular relating to further specificities, in addition to the two paratopes.

As used herein, the term "T cell redirecting" antibody refers to a (bispecific) antibody, which is able to "redirect" T cells to target cells, such as for example tumor cells. To this end, such a (bispecific) antibody typically has one specificity, e.g. one paratope, for recruiting T cells, which is preferably specific for T cells, preferably for T cell surface antigens, e.g. CD3. The other specificity, e.g. the other paratope, of such a "T cell redirecting" antibody is directed against a tumor-associated antigen, which may be selected depending on the target cell, e.g. a certain type of tumor cells. Such a "redirection" of a T cell to a target cell by a T cell redirecting, bispecific antibody typically results in T-cell mediated cell killing of the target cell.

Thus, the bispecific, in particular trifunctional, antibody for use in the treatment of cancer according to the present invention typically comprises a specificity against a tumor-associated antigen, to recruit tumor cells. Preferably, the tumor-associated antigen (or an epitope thereon, respectively) to be recognized by the bispecific, in particular trifunctional, antibody is selected from the group consisting of EpCAM, HER2/neu, HER3/neu, G250, CEA, MAGE, proteoglycans, VEGF, EGFR, .alpha.V.beta.3-integrin, HLA, HLA-DR, ASC, CD1, CD2, CD4, CD6, CD7, CD8, CD11, CD13, CD14, CD19, CD20, CD21, CD22, CD23, CD24, CD30, CD33, CD37, CD40, CD41, CD47, CD52, c-erb-2, CALLA, MHCII, CD44v3, CD44v6, p97, ganglioside GM1, GM2, GM3, GD1a, GD1b, GD2, GD3, GT1b, GT3, GQ1, NY-ESO-1, NFX2, SSX2, SSX4 Trp2, gp100, tyrosinase, Muc-1, telomerase, survivin, G250, p53, CA125 MUC, Wue antigen, Lewis Y antigen, HSP-27, HSP-70, HSP-72, HSP-90, Pgp, MCSP, EpHA2 and cell surface targets GC182, GT468 or GT512. It is also preferred that the tumor-associated antigen (or an epitope thereon, respectively) to be recognized by the bispecific, in particular trifunctional, antibody is selected from the group consisting of EpCAM, HER2/neu, HER3/neu, G250, CEA, MAGE, proteoglycans, VEGF, EGFR, .alpha.V.beta.3-integrin, HLA, HLA-DR, ASC, CD1, CD2, CD4, CD6, CD7, CD8, CD11, CD13, CD14, CD19, CD20, CD21, CD22, CD23, CD24, CD30, CD33, CD37, CD40, CD41, CD47, CD52, c-erb-2, CALLA, MHCII, CD44v3, CD44v6, p97, ganglioside GM1, GM2, GM3, GD1a, GD1b, GD2, GD3, GT1b, GT3, GQ1, NY-ESO-1, NFX2, SSX2, SSX4 Trp2, gp100, tyrosinase, Muc-1, telomerase, survivin, G250, p53, CA125 MUC, Wue antigen, Lewis Y antigen, HSP-27, HSP-70, HSP-72, HSP-90, Pgp, MCSP, EpHA2 and cell surface targets GC182, GT468, PD-L1, arboviral E protein (epitope) or GT512.

Preferably, the tumor-associated antigen (or an epitope thereon, respectively) to be recognized by the bispecific, in particular trifunctional, antibody is EpCAM. Preferably, the tumor-associated antigen (or an epitope thereon, respectively) to be recognized by the bispecific, in particular trifunctional, antibody is Her2/neu. Preferably, the tumor-associated antigen (or an epitope thereon, respectively) to be recognized by the bispecific, in particular trifunctional, antibody is GD2. Preferably, the tumor-associated antigen (or an epitope thereon, respectively) to be recognized by the bispecific, in particular trifunctional, antibody is GD3. Preferably, the tumor-associated antigen (or an epitope thereon, respectively) to be recognized by the bispecific, in particular trifunctional, antibody is CD20. Preferably, the tumor-associated antigen (or an epitope thereon, respectively) to be recognized by the bispecific, in particular trifunctional, antibody is CD19. Preferably, the tumor-associated antigen (or an epitope thereon, respectively) to be recognized by the bispecific, in particular trifunctional, antibody is CD30. Preferably, the tumor-associated antigen (or an epitope thereon, respectively) to be recognized by the bispecific, in particular trifunctional, antibody is CD33. Preferably, the tumor-associated antigen (or an epitope thereon, respectively) to be recognized by the bispecific, in particular trifunctional, antibody is PD-L1. Preferably, the tumor-associated antigen (or an epitope thereon, respectively) to be recognized by the bispecific, in particular trifunctional, antibody is EGF-R. Preferably, the tumor-associated antigen (or an epitope thereon, respectively) to be recognized by the bispecific, in particular trifunctional, antibody is an arboviral E protein epitope.

Accordingly, the bispecific, in particular trifunctional, antibody structure preferably encompasses one specificity, e.g. one paratope, which recognizes an epitope of a tumor antigen, preferably as described above, more preferably selected from EpCAM, GD2 and Her2, in particular Her2/neu, while its second specificity, e.g. its other paratope, recognizes an epitope of a T-cell surface antigen, preferably CD3.

For recruiting T cells and tumor cells by the bispecific, in particular trifunctional, antibody to be used according to the invention, the bispecific, in particular trifunctional, antibody to be used by the present invention therefore comprises another specificity, e.g. another paratope, which recognizes an epitope of a T cell surface receptor, preferably selected from the group consisting of CD3, CD2, CD4, CD5, CD6, CD8, CD28, CD40L and/or CD44, preferably CD3.

Therefore, the bispecific, in particular trifunctional, antibody for use in the treatment of cancer according to the present invention preferably binds by its first specificity, e.g. by its first paratope, to an epitope of the T-cell surface antigen, preferably CD3, and, by its second specificity, e.g. by its second paratope, to a tumor-associated antigen preferably selected from the group consisting of the tumor antigens EpCAM, HER2/neu, CD20 or the ganglioside GM1, GM2, GM3, GD1a, GD1b, GD2, GD3, GT1b, GT3 or GQ; more preferably, said bispecific, in particular trifunctional, antibody binds to CD3 by its first specificity, e.g. by its first paratope, and, by its second specificity, e.g. by its second paratope, to the tumor-associated antigen GD2; or alternatively preferably, said bispecific, in particular trifunctional, antibody binds to CD3 by its first specificity, e.g. by its first paratope, and, by its second specificity, e.g. by its second paratope, to the tumor antigen CD20; or alternatively preferably, said bispecific, in particular trifunctional, antibody binds to CD3 by its first specificity, e.g. by its first paratope, and, by its second specificity, e.g. by its second paratope, to tumor antigen EpCAM.

Preferably, a bispecific, in particular trifunctional, antibody to be used according to the invention is selected such that it exhibits, on the one hand, specificity to a T-cell surface antigen, e.g. CD3, and, on the other hand, specificity to a tumor-associated antigen, e.g. GD2 (anti-CD3 X anti-GD2) or CD20 (anti-CD3 X anti-CD20) or EpCAM (anti-CD3 x anti-EpCAM), or HER2 (anti-CD3 x anti-HER2) most preferably catumaxomab (anti-CD3 x anti-EpCAM), FBTA05/Lymphomun (anti-CD3 X anti-CD20), Ektomun (anti-CD3 X anti-GD2)or ertumaxomab (anti-CD3 x anti-HER2).

Preferably, a bispecific, in particular trifunctional, antibody to be used according to the present invention comprises one specificity, preferably one paratope, against CD3 and one specificity, preferably one paratope, against EpCAM (anti-CD3 x anti-EpCAM). Preferably, a bispecific, in particular trifunctional, antibody to be used according to the present invention comprises one specificity, preferably one paratope, against CD3 and one specificity, preferably one paratope, against Her2/neu (anti-CD3 x anti-Her2/neu). Preferably, a bispecific, in particular trifunctional, antibody to be used according to the present invention comprises one specificity, preferably one paratope, against CD3 and one specificity, preferably one paratope, against GD2 (anti-CD3 x anti-GD2). Preferably, a bispecific, in particular trifunctional, antibody to be used according to the present invention comprises one specificity, preferably one paratope, against CD3 and one specificity, preferably one paratope, against GD3 (anti-CD3 x anti-GD3). Preferably, a bispecific, in particular trifunctional, antibody to be used according to the present invention comprises one specificity, preferably one paratope, against CD3 and one specificity, preferably one paratope, against CD20 (anti-CD3 x anti-CD20). Preferably, a bispecific, in particular trifunctional, antibody to be used according to the present invention comprises one specificity, preferably one paratope, against CD3 and one specificity, preferably one paratope, against CD19 (anti-CD3 x anti-CD19). Preferably, a bispecific, in particular trifunctional, antibody to be used according to the present invention comprises one specificity, preferably one paratope, against CD3 and one specificity, preferably one paratope, against CD30 (anti-CD3 x anti-CD30). Preferably, a bispecific, in particular trifunctional, antibody to be used according to the present invention comprises one specificity, preferably one paratope, against CD3 and one specificity, preferably one paratope, against CD33 (anti-CD3 x anti-CD33). Preferably, a bispecific, in particular trifunctional, antibody to be used according to the present invention comprises one specificity, preferably one paratope, against CD3 and one specificity, preferably one paratope, against PD-L1 (anti-CD3 x anti-PD-L1). Preferably, a bispecific, in particular trifunctional, antibody to be used according to the present invention comprises one specificity, preferably one paratope, against CD3 and one specificity, preferably one paratope, against EGF-R (anti-CD3 x anti-EGF-R). Preferably, a bispecific, in particular trifunctional, antibody to be used according to the present invention comprises one specificity, preferably one paratope, against CD3 and one specificity, preferably one paratope, against an arboviral E protein epitope (anti-CD3 x anti- arboviral E protein).

As used herein, the term "trifunctional antibody" refers to a specific class of bispecific (or multispecific) antibodies, which has - in addition to the two (or more) paratopes and the resulting ability to bind to two (or more) different epitopes on the same or different antigens - a third "functionality". Preferably, such a third "functionality" is different from a (third) "specificity", i.e. although the "third functionality" may be a binding site, it is preferably not an antigen binding site. In other words, the "third functionality" is preferably not a paratope. Preferred examples for such a "third functionality" include a binding site for an Fc receptor, the ability to activate or stimulate immune cells or/and induction of anti-tumor responses or anti-tumor immunity. Thus, trifunctional antibodies which may be preferably employed according to the present invention, typically exhibit three functions, in particular including their ability to bind (redirect) T-cells and to bind (target) tumor cells by the two specificities (antigen binding sites), and - as a third function - trifunctional antibodies are in particular able to stimulate/activate immune cells as e.g. T cells or/and accessory cells demonstrated by e.g the release of pro-inflammatory cytokines (as e.g. IL-6, TNF-alpha, IFN-gamma or/and IL8) or/and the upregulation of costimulatory molecules on T-cells or/and accessory cells as e.g. CD25, CD28, CD40, CD69, CD80 or/and CD86.

The bispecific trifunctional antibody as used according to the present invention comprises an Fc region (fragment crystallizable region, also referred to as "Fc portion"). As used herein, the term "Fc region" refers to a sequence derived from the portion of an immunoglobulin heavy chain beginning in the hinge region just upstream of the papain cleavage site and ending at the C-terminus of the immunoglobulin heavy chain. Preferably, the "Fc region" comprises a binding site for an Fc receptor. However, it is also preferred that an Fc region may mediate a functionality different from binding to an Fc receptor, for example binding to a protein of the complement system. Accordingly, an "Fc region" may be a complete Fc region or a part (e.g., a domain) thereof. Preferably, the "Fc region" mediates the full functionality of a complete Fc region, e.g. including Fc receptor binding and, optionally, binding to a protein from the complement system. Thus, the antibody as used according to the present invention preferably comprises a complete Fc region, whereby a complete Fc region comprises at least a hinge domain, a CH2 domain, and a CH3 domain. The Fc region may also comprise one or more amino acid insertions, deletions, or substitutions relative to a naturally-occurring Fc region. For example, at least one of a hinge domain, CH2 domain or CH3 domain (or portion thereof) may be deleted. For example, an Fc region may comprise or consist of: (i) hinge domain (or portion thereof) fused to a CH2 domain (or portion thereof), (ii) a hinge domain (or portion thereof) fused to a CH3 domain (or portion thereof), (iii) a CH2 domain (or portion thereof) fused to a CH3 domain (or portion thereof), (iv) a hinge domain (or portion thereof), (v) a CH2 domain (or portion thereof), or (vi) a CH3 domain or portion thereof.

The bispecific, in particular trifunctional, antibody used according to the present invention comprises an Fc region. Accordingly, it is preferable, that a bispecific, in particular trifunctional, antibody to be used according to the present invention comprises one specificity, preferably one paratope, against CD3; one specificity, preferably one paratope, against EpCAM (anti-CD3 x anti-EpCAM); and an Fc region. Preferably, a bispecific, in particular trifunctional, antibody to be used according to the present invention comprises one specificity, preferably one paratope, against CD3; one specificity, preferably one paratope, against Her2/neu (anti-CD3 x anti-Her2/neu); and an Fc region. Preferably, a bispecific, in particular trifunctional, antibody to be used according to the present invention comprises one specificity, preferably one paratope, against CD3; one specificity, preferably one paratope, against GD2 (anti-CD3 x anti-GD2); and an Fc region. Preferably, a bispecific, in particular trifunctional, antibody to be used according to the present invention comprises one specificity, preferably one paratope, against CD3; one specificity, preferably one paratope, against GD3 (anti-CD3 x anti-GD3); and an Fc region. Preferably, a bispecific, in particular trifunctional, antibody to be used according to the present invention comprises one specificity, preferably one paratope, against CD3; one specificity, preferably one paratope, against CD20 (anti-CD3 x anti-CD20); and an Fc region. Preferably, a bispecific, in particular trifunctional, antibody to be used according to the present invention comprises one specificity, preferably one paratope, against CD3; one specificity, preferably one paratope, against CD19 (anti-CD3 x anti-CD19); and an Fc region. Preferably, a bispecific, in particular trifunctional, antibody to be used according to the present invention comprises one specificity, preferably one paratope, against CD3; one specificity, preferably one paratope, against CD30 (anti-CD3 x anti-CD30); and an Fc region. Preferably, a bispecific, in particular trifunctional, antibody to be used according to the present invention comprises one specificity, preferably one paratope, against CD3; one specificity, preferably one paratope, against CD33 (anti-CD3 x anti-CD33); and an Fc region. Preferably, a bispecific, in particular trifunctional, antibody to be used according to the present invention comprises one specificity, preferably one paratope, against CD3; one specificity, preferably one paratope, against PD-L1 (anti-CD3 x anti-PD-L1); and an Fc region. Preferably, a bispecific, in particular trifunctional, antibody to be used according to the present invention comprises one specificity, preferably one paratope, against CD3; one specificity, preferably one paratope, against EGF-R (anti-CD3 x anti-EGF-R); and an Fc region. Preferably, a bispecific, in particular trifunctional, antibody to be used according to the present invention comprises one specificity, preferably one paratope, against CD3; one specificity, preferably one paratope, against an arboviral E protein epitope (anti-CD3 x anti- arboviral E protein); and an Fc region.

Preferably, the bispecific, in particular trifunctional, antibody to be used according to the invention is an IgG type antibody comprising an Fc region. More preferably, a trifunctional bispecific antibody to be used according to the invention is a heterologous intact rat/mouse antibody comprising an Fc region, whereby an antibody with a subclass combination of mouse IgG2a and rat IgG2b is preferred. A heterologous intact rat/mouse antibody comprising an Fc region, with a heavy chain composed of murine IgG2a and rat IgG2b subclasses, each with their respective light chains, is particularly preferred.

In general, a trifunctional bispecific antibody to be used according to the invention exhibits preferably one of the following isotype combinations in its Fc-region: rat-IgG2b/mouse-IgG2a, rat-IgG2b/mouse-IgG2b, rat-IgG2b/human-IgG1, or mouse-[VH-CH1,VL-CL]-human-IgG1/rat-[VH-CH1,VL-CL]-human-IgG1-[hinge]-human IgG3*-[CH2-CH3], wherein * = caucasian allotypes G3m(b+g) = no binding to protein A.

The Fc region enables the trifunctional antibody to additionally recruit cells expressing an Fc receptor, such as Fcγ receptor positive accessory cells, for example macrophages, dendritic cells, natural killer (NK) cells, and other Fc-receptor-expressing cells. Since trifunctional antibodies are bispecific (or multispecfic) antibodies, they are preferably able to recruit and activate (i) T cells and (ii) Fc-receptor expressing cells, such as accessory immune cells, for example monocytes/macrophages, natural killer cells, dendritic cells or other Fc receptor expressing cells, simultaneously at the (iii) targeted tumor by their anti-tumor specificity. The simultaneous activation of these different classes of effector cells results in efficient killing of the tumor cells by various mechanisms such as, for example, phagocytosis and perforin-mediated cytotoxicity. Typically, the net effect of a preferred trifunctional antibody, which comprises an Fc receptor, is linking T cells and Fc receptor positive cells to target cells, e.g. tumor cells, leading to the destruction of the tumor cells. Trifunctional antibodies evoke the removal of tumor cells by means of (i) antibody-dependent cell-mediated cytotoxicity, (ii) T-cell mediated cell killing, and (iii) induction of anti-tumor immunity. The inventors of the present invention have surprisingly identified a concept to overcome severe inflammatory reactions observed by prior art studies (e.g. employing bispecific, in particular trifunctional, antibodies) to reduce and/or suppress such inflammatory reactions by subcutaneously administering bispecific, in particular trifunctional, antibodies. The reduction, preferably the almost total avoidance, of the release of inhibitory cytokines, such as IL-10, occurring in a mammal, typically after intravenous or intraperitoneal administration of the T-cell redirecting bispecific, in particular trifunctional, antibodies, paves the way for a new era in cancer immunotherapy. The efficacy of anti-cancer therapy by bispecific, in particular trifunctional, antibodies may be decisively enhanced by the present invention, as inhibitory mechanisms to cell-mediated immunity, as observed so far, are reduced or eliminated by selecting the subcutaneous administration route.

Additionally, subcutaneous administration of the bispecific, in particular trifunctional, antibody is also expected to provoke a depot effect. By the subcutaneous route, the bispecific, in particular trifunctional, antibodies are expected to be slowly released from the subcutaneous depot into the intravascular space. Hereby, peak serum concentrations are avoided, which otherwise occur upon e.g. intravenous administration. Accordingly, a balanced steady-state serum concentration of the administered antibody is established by subcutaneous administration. That mode of administration may even allow for administering higher amounts of bispecific, in particular trifunctional, antibodies as compared to e.g. the intravenous route, without, however, triggering adverse side-effects. In terms of cancer treatment, higher levels of administered antibodies in the circulation of the patient for a prolonged period of time enhance the efficacy of the treatment.

The term "subcutaneous administration" (also referred to as "s.c." administration) within the meaning of the present invention refers to introduction, e.g. by injection, of the bispecific, in particular trifunctional, antibody under the skin, namely of an animal or human patient. The target area of administration can be the fat layer located between the dermis and underlying fascia. Usually, the term "subcutaneous administration" as used herein refers to a delivery into the interstitial space of the hypodermis, also referred to as subcutaneous tissue. The hypodermis is located between the skin and the deep fascia covering the muscle tissue. The hypodermis consists mainly of adipose tissue, whereby cellular components of the hypodermis include mainly adipocytes, e.g. in the adipose tissue lobes, and, to a lesser extent, macrophages and fibroblasts, with the latter being present, e.g., in connective tissue septa and synthesizing components of the extracellular matrix (ECM). In general, subcutaneous administration is to be distinguished from interdermal administration, which refers to a delivery into the dermis and/or epidermis. After s.c. administration antibodies can be transported to the systemic circulation directly via blood capillaries or indirectly via lymphatics, both of which contribute to the absorption of the antibodies from the SC interstitial region. Absorption is also affected by transport through ECM and presystemic elimination. Details regarding the absorption of biotherapeutics in general following s.c. administration can be retrieved from Richter et al. (Richter et al. (2012) The AAPS Journal 14 (3): 559-567). The introduction of the bispecific, in particular trifunctional, antibody can be carried out by creating a pocket between the skin and the underlying tissue. The pocket may be created by pinching or drawing the skin up and away from underlying tissue.

Sites for administering the antibody subcutaneously may include the outer area of the upper arm, the thoracic region, in particular the lower thoracic region, the abdominal wall, above or below the waist, the upper area of the buttock, just behind the hip bone and the thigh, in particular the front of the thigh. Preferred sites for administering the antibody subcutaneously include the abdominal wall, the lower thoracic region, and the thigh. Within a treatment cycle, each single dose may be administered to essentially the same body site, e.g. the thigh or abdomen. Alternatively, each single dose within a treatment cycle may be administered to different body sites.

A "single" dose dose of the antibody or of the respective pharmaceutical composition may be administered in particular by one single or by several, preferably by 1, 2, 3, 4 or 5 administrations (e.g. injections), wherein such several administrations are either performed simultaneously or in immediate succession in order to represent a "single" dose. Thus, the administration of the single dose (i.e. administration of all of the optionally several applications/injections in total) takes preferably no longer than 1 hour, more preferably no longer than 30 min, even more preferably no longer than 10 min. By administration of a single dose via several, preferably 2, 3, 4, or 5, administrations, e.g. injections, subcutaneous administration of large volumes can be avoided. Preferably, the bispecific, in particular trifunctional, antibody is administered subcutaneously in liquid form. The volume of the liquid for administering the bispecific, in particular trifunctional, antibody is typically up to 5 ml for a single administration (usually corresponding to a single dose), preferably up to 3 ml, more preferably up to 2 ml, even more preferably up to 1 ml.

For subcutaneous administration different devices may be used, e.g. a syringe (including a pre-filled syringe); an injection device (e.g. the INJECT-EASET™ and GENJECTT™ device); an infusion pump (such as e.g. Accu-Chek™); an injector pen (such as the GENPENT™); a needleless device (e.g. MEDDECTOR™ and BIOJECTOR™); an autoinjector; or a subcutaneous patch delivery system. The devices which may preferably be used for subcutaneous injection may include syringes with preferably short, e.g. 5/8-inch-long, needles as described in the following.

The width (diameter, in particular outer diameter) of a needle for subcutaneous administration is typically 25 or 27 gauge or, preferably, greater than or equal to 27 gauge. More preferably, the diameter, in particular the outer diameter, of a needle for subcutaneous administration is at least 28 gauge. Even more preferably, the diameter, in particular the outer diameter, of a needle for subcutaneous administration (e.g. injection) is at least 29 gauge, for example 29 gauge, 29 ½ gauge, 30 gauge, 30 5/16 gauge, or 31 gauge. Particularly preferably, the diameter, in particular the outer diameter, of a needle for subcutaneous administration is at least 30 gauge. The use of such needles having very small outer diameters is assumed to further modify the cytokine release, possibly by causing smaller lesions and/or by causing a slower administration (less volume released over the same time). Needle injection typically requires injection by positioning the needle at an angle withing the range of 40 to 50°.

Subcutaneous introduction of the bispecific, in particular trifunctional, antibody may also be enabled by needle-free injection or a meter dose infusion pump. Such infusion pumps may be selected from CADD-PCA®, Braun Perfusor M® or ME®, Braun Perfusor Compact® or LogoMed Pegasus PCA®. Alternatively, the bispecific, in particular trifunctional, antibody may also be provided for subcutaneous administration in a "pen" where a short one-use needle is screwed on to the end of a pen-shaped, multi-use vial. The amount of medication needed is then dialed in at the end. Typically, the bispecific, in particular trifunctional, antibody is provided by a liquid pharmaceutical composition as detailed below when administered subcutaneously.

Within the context of this invention the term "dose" refers to the amount (quantity) of antibody administered, e.g. the amount of antibody given by a single administration. The term "administration", as used herein, means introduction of the bispecific, in particular trifunctional, antibody into a mammal by subcutaneous administration as described herein, e.g. by subcutaneous injection, e.g. a single bolus injection.

The treatment protocol according to the present invention is typically composed of a number of single administrations which form a treatment cycle. The patient may be subjected to one single or various treatment cycles. Each treatment cycle is typically composed of from 2 to 28, preferably from 2 to 20, more preferably from 5 to 15, and even more preferably from 6 to 10 individual subcutaneous doses. Preferably, within a treatment cycle (starting with an initial dose and ending with a final dose), the dose of each single administration (except the initial dose) is not lower than the previous dose administered, i.e. each subsequent dose is equal to or higher than the previous one, more preferably each subsequent dose is higher than the previous one, e.g. dosing may be increased (starting from the initial dosing) by a factor of from 1.5 to 5 for each subsequent dose as compared to the previous dose, preferably from 1.5 to 3, more preferably from 1.5 to 2.5, until a maximum dosage is reached. However, such increased dosing may optionally also include doses which are equal to the previous one. Thereby, preferably the majority of single doses is higher than the previous one while no single dose is lower than the previous one. Whenever the repeating dose is a second dose, the previous dose is the initial dose ("first dose" or "starting dose"). Where the repeating dose is a third dose, the previous dose is the second dose. Thus, the term "previous dose" within the meaning of the present application refers to the dose immediately preceeding the current dose to be administered to the patient, said patient being diagnosed with at least one type of cancer.

Typically, a single treatment cycle includes at least an initial (first) dose and a second dose. In a preferred embodiment a single treatment cycle may include an initial (first) dose, a second dose, a third dose, a fourth dose, a fifth dose and preferably, additionally a sixth dose. Within a single treatment cycle a repeating dose may be typically administered 1, 2, 3, 4, 5, 6, or 7 days after the previous dose, preferably the repeating dose is administered 1 - 5 days after the previous dose, more preferably the repeating dose is administered 1 - 4 days after the previous dose, even more preferably the repeating dose is administered 1 - 3 days after the previous dose, and most preferably the repeating dose is administered about 1 day after the previous dose. In other words, it is particularly preferred that the bispecific, in particular trifunctional, antibody is administered daily, preferably a single dose per day is administered via the subcutaneous route. Thereby, a frequent, preferably daily, administration of the antibody is assumed to support a rapid and complete depletion of the patient's B-cell population and, thereby, unwanted HAMA (human anti-mouse antibody) or ADA (anti-drug antibody) reactions due to only partial depletion of the patient's normal B-cell population can be avoided. The HAMA response is a reaction to mouse antibodies that can range from mild forms, like a rash, to a more extreme response, such as renal failure (cf. Julius M. Cruse, Robert E. Lewis: Illustrated Dictionary of Immunology, Third Edition, 2009, CRC Press - "HAMA"). HAMA or ADA can also decrease the effectiveness of the treatment once the patient is re-treated by mouse antibodies.

At the end of any treatment cycle one, two or more subcutaneous administrations reflecting the maximum dosing may be foreseen.

The total dose (as the sum of each single dose within a treatment cycle) of bispecific, in particular trifunctional, antibody administered subcutaneously per treatment cycle will typically be from 1000 µg to 100000 µg, preferably from 1000 µg to 50000 µg, more preferably from 5000 µg to 10000 µg.

The inventors of the present invention found that - by subcutaneous application - amounts for one single dose (maximum dosing) of up to 10000 µg, preferably of up to 3000 µg, of bispecific, in particular trifunctional, antibodies as defined herein (e.g. as a maximum dose within a treatment cycle) may be administered without evoking adverse reactions. In contrast, intravenous administration of bispecific, in particular trifunctional, antibodies exhibit inflammatory reactions and other side effects at doses as low as 200 µg (Kiewe et al. (2006) Clin Cancer Res, 12: 3085-3091). Accordingly, such a higher dosing according to the present invention is highly promising for cancer therapy, while the patients treated do not develop symptoms of cytokine release syndrome with pyrexia and sometimes rigors or nausea, vomiting, headache, pain, tachycardia, elevated liver enzymes, arthralgia, systolic hypertension, fever and lymphocytopenia as previously observed in the art when administering bispecific, in particular trifunctional, antibodies at lower doses.

Not only the maximum dosing within a treatment cycle is critical, but also the initial closing of each treatment cycle. It should be noted that the terms "first dose", "initial dose" and "starting dose" are used within the meaning of the present invention interchangeably. Amounts as low as 5 µg or 10 µg of bispecific, in particular trifunctional, antibodies; e.g. catumaxomab, FBTA05/Lymphomun or ertumaxomab (Kiewe et al. (2006) Clin Cancer Res, 12: 3085; Burges et al. (2007) Clin Cancer Res, 13: 3899) were employed as the starting dose for cancer therapy in the art. Low amounts, in particular low amounts of the initial doses, were considered to be required to reduce the pronounced potential of bispecific, in particular trifunctional, antibodies to activate T cells leading to a release of proinflammatory cytokines, such as IL-2, IFN-gamma or TNF-α and the upregulation of costimulatory molecules like e.g. CD28.

However, the inventive finding based on the avoidance of the release of such cytokines by subcutaneous administration allows to increase the starting dose considerably. 20 to 100 µg or even more than 100 µg (e.g. between 100 and 300 µg, for example 200 µg) of bispecific, in particular trifunctional, antibodies may be administered as the initial dose of each treatment cycle. Such an increased initial dosing is not only well tolerated by cancer patients being subjected to subcutaneous administration, but also allows for establishing a significantly more aggressive cancer treatment. The inventive finding allowing for increasing the initial dosing also addresses another issue: Low starting doses are also less desired, as they may induce unwanted HAMA (human anti-mouse antibody) reactions due to only partial depletion of the patient's normal B-cell population at the beginning of the treatment regimen with bispecific, in particular trifunctional, antibodies, such as FBTA05. The HAMA response is a reaction to mouse antibodies that can range from mild forms, like a rash, to a more extreme response, such as renal failure (cf. Julius M. Cruse, Robert E. Lewis: Illustrated Dictionary of Immunology, Third Edition, 2009, CRC Press - "HAMA"). HAMA can also decrease the effectiveness of the treatment once the patient is re-treated by mouse antibodies.

Administration of the first (or "initial" or "starting") dose is preferably carried out at an amount of up to 500 µg, in particular of up to 300 µg, e.g. at an amount from 10 µg - 500 µg, preferably at an amount of from 30 µg to 200 µg, more preferably from 40 µg to 150 µg, even more preferably from 40 µg to 120 µg, most preferably from 40 µg to 100 µg, e.g. from 40 µg to 60 µg, of the bispecific, in particular trifunctional, antibody as described herein. Administration of the second dose within the treatment cycle is typically increased as compared to the initial dose and may be chosen from an amount of between 100 µg and 800 µg, preferably from 100 µg to 500 µg, more preferably from 100 µg to 300 µg of antibody. The administration of a third dose is performed in an amount of up to 1000 µg, preferably of up to 700 µg, more preferably from 200 µg to 600 µg of antibody. Typically, the dosing is increased from the previous subcutaneous introduction to the subsequent one within a treatment cycle by a factor of 1.5 to 3, preferably by a factor of 1.5 to 2.5, which may even be increased in some instances (requiring a more aggressive treatment protocol) to a dosing of up to 5 times of the previous dosage.

The administration of a maximum dose, which may be given once, twice or more often at the end of each treatment cycle, may be chosen from an amount of up to 10000 µg, preferably of up to 7500 µg, more preferably of up to 5000 µg, even more preferably of up to 3000 µg, preferably 800 µg or more, more preferably 1000 µg or more, even more preferably 1500 µg or more, particularly preferably 2000 µg or more, of the bispecific, in particular trifunctional, antibody as disclosed herein.

Accordingly, a dosage regimen for administering the bispecific, in particular trifunctional, antibody as described herein to a human patient diagnosed with cancer may comprise: (a) subcutaneously administering an initial (first or starting) dose of said antibody; and consecutively (b) subcutaneously administering at least one repeating dose of said antibody; wherein said at least one repeating dose equals or exceeds the previous dose at a factor of 1.5 to 5, preferably 1.5 to 3, more preferably 1.5 to 2.5, of the amount administered as previous dose.

The dosage regimen includes at least one repeating dose. Preferably, the dosage regimen includes an initial (first) dose and a second dose. In a preferred embodiment the dosage regimen could include an initial (first) dose, a second dose, a third dose, a fourth dose, a fifth dose and preferably, additionally a sixth dose.

In general, the dosage regimen is reflected by the treatment protocol. A treatment protocol comprises all treatment cycles, i.e. a treatment protocol may include one or more treatment cycles as outlined below, whereby one treatment cycle usually includes one or more single doses, preferably more than one single dose.

Thus, in one preferred embodiment the initial dose may be 10 µg, preferably 50 µg, followed by a second dose of 100 µg, a third dose of 200 µg, a fourth dose of 400 µg, a fifth dose of 600 µg and a sixth dose of 800 µg, whereby the treatment starting with the initial dose and ending with the sixth dose is one treatment cycle in this embodiment. In another preferred embodiment the initial dose may be 100 µg, followed by a second dose of 200 µg, a third dose of 400 µg, a fourth dose of 800 µg, a fifth dose of 1000 µg, a sixth dose of 1500 µg, and a seventh dose of 2000 µg.

Typically, each (single) dose within one treatment cycle is preferably administered within 1 hour, more preferably within 30 minutes, even more preferably within 10 minutes, and particularly preferably between 5 minutes, e.g. by injection or infusion. For example, each dose within one treatment cycle is administered within 1 to 30 minutes, preferably within 1 to 10 minutes, more preferably between (i.e. within) 1 to 5 minutes, e.g. by injection or infusion.

The bispecific, in particular trifunctional, antibody as defined herein is typically used in a treatment of cancer wherein said treatment preferably encompasses one single or repeated treatment cycles. Each treatment cycle may be repeated once, twice, three times or more often. Typically, a treatment cycle of e.g. 6 to 10 doses to be administered every 1 to 4 days, e.g. every 3 to 4 days, will last for a period of between 18 to 40 days. Several treatment cycles are typically interrupted by treatment-free periods of between 2 to 6 weeks, typically, 2 to 4 weeks. Thereafter, the treatment may be repeated by another treatment cycle which may essentially be identical to the previous treatment cycle (identical dosing and administration protocol). The dosing of the second, third and any further treatment cycle may alternatively also be modified as compared to the previous treatment cycle (depending on the effects observed in the course of the previous cycle). Typically, the dosing of each single administration of the second and third treatment cycle is either the same or slightly (e.g. by up to 30%) increased over the initial dosing. At the end of the treatment protocol, e.g. by the fourth, fifth or sixth treatment cycle of the dosing of each single administration may be lowered again, e.g. to reflect the dosing of or even less than the first cycle.

The bispecific, in particular trifunctional, antibodies used according to the invention may be modified such that improved effects are observed. The subcutaneous depot effect may e.g. be prolonged with modifications of the bispecific, in particular trifunctional, antibody to alter its hydrophobicity. For this purpose biocompatible polymers such as polyethylene glycol (PEG) may be employed by covalently attaching one or more PEG molecules to said antibody; i.e. PEGylation. Hereby, agglomeration in the serum is avoided and their in vivo half-life is prolonged.

The bispecific, in particular trifunctional, antibody used in accordance with the present invention is typically applied as a pharmaceutical composition. Accordingly, the pharmaceutical composition comprising the bispecific, in particular trifunctional, antibodies is typically administered according to the treatment protocol described herein. Accordingly, preferred embodiments of the bispecific, in particular trifunctional, antibody, as described herein, are also applicable for the pharmaceutical composition; regarding for example the specificity of the antibody, the disease to be treated, and the administration (e.g., the dose of the antibody, the treatment protocol, and/or the performance of the s.c. application). The "pharmaceutical composition" relates to a composition containing the bispecific, in particular trifunctional, antibody for subcutaneous administration to a mammal. Thus, the present invention provides in a further aspect a pharmaceutical composition for use in the treatment of a cancer disease, wherein said pharmaceutical composition comprises a bispecific, in particular a T cell redirecting, trifunctional, antibody, wherein said composition is administered subcutaneously. Preferred embodiments of the pharmaceutical composition refer to preferred embodiments of the bispecific antibody for use according to the present invention as described herein.

The pharmaceutical composition of the present invention may be provided as a lyophilized powder or, more preferably in solution (dissolved in a vehicle). If provided as lyophilized powder by the manufacturer, it is usually dissolved in an appropriate solution (aqueous solution) shortly prior to subcutaneous administration. Such a powder is to be reconstituted with an appropriate liquid vehicle, which may be water for injection or isotonic saline, optionally buffered. Vials of liquid medication can be single use or multi-use.

In another preferred embodiment, the antibody and/or the pharmaceutical composition for use according to the present invention is not lyophilized. Thus, it is preferred that the bispecific, in particular trifunctional, antibody for use according to the present invention is not lyophilized, but provided in a solution, preferably in an aqueous solution, more preferably in an aqueous buffered solution.

The pharmaceutical composition as used according to the present invention may furthermore comprise, in addition to one of these substances, a (compatible) pharmaceutically acceptable carrier, excipient, buffer, stabilizer or other materials well known to those skilled in the art. In this context, the expression "(compatible) pharmaceutically acceptable carrier" preferably includes the liquid or non-liquid basis of the composition. The term "compatible" means that the constituents of the pharmaceutical composition as used herein are capable of being mixed with the pharmaceutically active component, i.e. the antibody, as defined above and with one other component in such a manner that no interaction occurs which would substantially reduce the pharmaceutical effectiveness of the composition under usual use conditions. Pharmaceutically acceptable carriers must, of course, have sufficiently high purity and sufficiently low toxicity to make them suitable for administration to a person to be treated.

If the pharmaceutical composition as used herein is provided in liquid form, the pharmaceutically acceptable carrier will typically comprise one or more (compatible) pharmaceutically acceptable liquid carriers. The composition may comprise as (compatible) pharmaceutically acceptable liquid carriers e.g. pyrogen-free water; isotonic saline or buffered (aqueous) solutions, e.g. phosphate or citrate buffered solutions, vegetable oils, such as, for example, groundnut oil, cottonseed oil, sesame oil, olive oil, corn oil and oil from theobroma; polyols, such as, for example, polypropylene glycol, glycerol, sorbitol, mannitol and polyethylene glycol; alginic acid.

Thus, preferred ingredients of the composition in solution may be stabilizing agents such as Tween® 80, Tween® 20 and/or buffer compounds, e.g. sodium citrate buffer. Particularly for injection of the pharmaceutical composition as used herein, a buffer, preferably an aqueous buffer, may be used.

Preferably the pharmaceutical composition for use according to the present invention thus comprises a buffer. The buffer is preferably based on an organic acid. Thus, the pharmaceutical composition for use according to the present invention preferably comprises a buffer, more preferably an organic acid buffer. The organic acid buffer is preferably selected from the group consisting of citrate buffer, phosphate buffer, succinate buffer, tartrate buffer, buffered saline, and phosphate-citrate buffer, more preferably selected from the group consisting of citrate buffer, phosphate buffer, succinate buffer and tartrate buffer. It is particularly preferred that the buffer is a citrate buffer, whereby a sodium-citrate buffer is most preferred.

The pharmaceutical composition for subcutaneous administration may be obtained by diluting a concentrate containing the antibody in an appropriate liquid vehicle for administration, whereby a preferred appropriate liquid vehicle is isotonic saline, i.e. 0.9 % sodium chloride solution. The concentrate containing the antibody may be formulated as a stable, sterile, preferably preservative-free and preferably pyrogen-free liquid pharmaceutical formulation comprising from 0.05 to 5 mg/ml, preferably 0.05 to 3 mg/ml, antibody in aqueous solution, preferably aqueous buffered solution, more preferably citrate buffer, even more preferably sodium citrate buffer, most preferably 20 - 100 mM, e.g. 100 mM, sodium citrate buffer, whereby the aqueous solution, aqueous buffered solution, or sodium citrate buffer, respectively, is preferably at pH 5.6 - 7.5. Namely, the concentration of the buffer, preferably of the organic acid buffer as described herein, more preferably of the citrate buffer, even more preferably of the sodium citrate buffer, in the pharmaceutical composition for use according to the present invention is preferably no more than 100 mM, more preferably no more than 50 mM, even more preferably no more than 40 mM and particularly preferably no more than 30 mM, e.g. 20 mM. Moreover, the pH value of the pharmaceutical composition of the pharmaceutical composition for use according to the present invention is preferably from 5.5 to 7.5, more preferably from 5.6 to 7.4, even more preferably from 5.7 to 6.3, and most preferably from 5.8 to 6.2, e.g. 6.0. It is assumed that such buffer concentrations and/or pH values further modify, in particular reduce, the cytokine release upon subcutaneous administration of the antibody.

The concentrate may contain a stabilizer, e.g. Tween® 80 at a concentration of e.g. 0.02% (w/v). Preferably, the concentrate is provided in prefilled syringes with a single syringe containing a predetermined amount of antibody, e.g. a single syringe containing 5, 10, 20, 40, 50, 100, 200, 500, 1000, 3000 or 5000 µg of the antibody, preferably 10, 50 or 100 µg of the antibody, more preferably 50 µg of the antibody. The concentrate may be dissolved in an appropriate liquid vehicle for administration, preferably in isotonic saline, for administration depending on the dose level.

Additionally, the pharmaceutical composition for subcutaneous administration may also contain excipients to facilitate the administration via the subcutaneous route. Furthermore, if larger volumes are administered, hyaluronidase may be co-administered, i.e. in the pharmaceutical composition according to the present invention or in a separate composition, which is co-administered with the pharmaceutical composition according to the present invention. Co-administration of hyaluronidase allows the administration of larger volumes via the s.c. route by transient cleavage of hyaluronan.

Preferably, the antibody to be used according to the invention may be administered in combination with a glucocorticoid, in particular with a naturally occurring glucocorticoid, e.g. hydrocortisone (cortisol) or cortisone, or with a synthetic glucocorticoid. Preferred examples of glucocorticoids include prednisone, prednisolone, methylprednisolone, Triamcinolone, Betamethasone, Dexamethasone, Cortisonacetate, Prednylidene, Deflazacorte, Cloprednol, Fluocortolone und Budenoside. Preferably, the glucocorticoid is hydrocortisone (cortisol). Preferably, the glucocorticoid is cortisone. Preferably, the glucocorticoid is dexamethasone. Preferably, the glucocorticoid is betamethasone. Preferably, the glucocorticoid is administered topically, preferably at the site of the subcutaneous antibody administration (e.g., injection). For example, it is particularly preferred that the site of antibody/pharmaceutical composition administration (e.g. injection site) is treated for example with a glucocorticoid cream, e.g. preferably hydrocortisone (cortisol) cream or cortisone cream, preferably (immediately) after each antibody administration (e.g. injection). It is also preferred that the site of antibody/pharmaceutical composition administration (e.g. injection site) is treated for example with a glucocorticoid cream, e.g. preferably dexamethasone cream or betamethasone cream, preferably (immediately) after each antibody administration (e.g. injection). Thus, when used in combination, the bispecific, in particular trifunctional, antibody and the glucocorticoid are preferably administered separately. Thereby administration of the glucocorticoid up to one hour before, concomitantly, or up to one hour after administration of the bispecific, in particular trifunctional, antibody is preferred and administration of the glucocorticoid up to 30 min after administration of the bispecific, in particular trifunctional, antibody is particularly preferred.

Thus, the bispecific antibody or the pharmaceutical composition for use according to the present invention is preferably administered in combination with a glucocorticoid, wherein the glucocorticoid is preferably administered topically at the site of antibody administration. Moreover, the present invention also provides in a further aspect a combination therapy for use in the treatment of a cancer disease, wherein the bispecific antibody or the pharmaceutical composition according to the present invention are administered subcutaneously in combination with with a glucocorticoid, wherein the glucocorticoid is preferably administered topically at the site of antibody administration. It is assumed that such combination therapy with glucocorticoids further modifies, in particular reduces, the cytokine release upon subcutaneous administration of the antibody.

The antibody to be used according to the invention may be employed for any cancer disease, in particular the cancer diseases disclosed herein, e.g. non-solid cancer diseases, e.g. leukemia or lymphoma, or solid cancers. The treatment may be a monotherapeutic treatment, which exclusively employs the bispecific, in particular trifunctional, antibody as an active anti-cancer drug. However, the intentive method may also be employed prior to, parallel to or subsequent to the administration of other agents, e.g. anti-cancer agents, e.g. as a component of a combination therapy (e.g. combined therapy of an anti-cancer drug and a bispecific, in particular trifunctional, antibody). Accordingly, said bispecific, in particular trifunctional, antibody may be concomitantly administered together with an anticancer agent to an individual diagnosed with cancer. Said anti-cancer agent (anti-cancer drug) may be a small organic compound, e.g. methotrexate, taxol, hydroxycarbamide, irinotecane, mitotan, cyclizin, etoposide, topotecane, procarbazine, bortezomib, omacetaxine, cladribine and bleomycin, doxorubicine, idarubicine, daunorubicine, cisplatin, oxaliplatin, busulfan, ifosfamide, melphalane, cyclophosphamide or anti-cancer cytokines (e.g. IFN). The anti-cancer drug may also be an anti-cancer antibody (e.g. a monoclonal (monospecific) antibody) being directed to a tumor antigen, e.g. selected from the group consisting of trastuzumab, alemtuzumab, bevacizumab, brentuximab vedotin, cetuximab, gemtuzumab ozogamicin, ibritumomab tiuxetan, ipilmumab, nivolumomab, nimotuzumab, ofatumumab, panitumumab, rituximab and tositumomab. Preferably, the anti-cancer drug may also be an anti-cancer antibody selected from the group consisting of nivolumab, pembrolizumab, blinatumomab. Typically, the bispecific, in particular trifunctional, antibody and said anti-cancer drug are not provided as a combination preparation, but administered separately, typically by distinct formulations. The formulations may be administrated by separate administration routes, e.g. the bispecific, in particular trifunctional, antibody may be administered subcutaneously, while the small organic anti-cancer compound or the anti-cancer monoclonal monospecific antibody may be administered by any other administration route, e.g. orally, subcutaneously or intravenously.

The bispecific, in particular trifunctional, antibody administered subcutaneously according to the invention may also be used as a component of a combination therapy for the treatment of a cancer disease in combination with a corticosteroid compound. Such a corticosteroid compound may be preferably selected from the betamethasone group, e.g. dexamethasone, betamethasone or fluocortolone. It may be administered intravenously or by any other appropriate route of administration.

Thus, the bispecific, in particular trifunctional, antibody administered subcutaneously according to the invention is preferably administered in combination with a glucocorticoid as described herein, wherein the glucocorticoid is preferably administered topically, preferably at the site of (subcutaneous) administration of the bispecific, in particular trifunctional, antibody, and, preferably, in combination with an anti-cancer agent, preferably a small organic compound or an anticancer antibody as described herein, wherein the anticancer agent is preferably administered intravenously or subcutaneously.

The cancer disease to be treated by the bispecific antibody, in particular trifunctional, antibody according to the present invention and/or with the pharmaceutical composition according to the present invention may be selected from the group consisting of solid or non-solid tumors, preferably selected from the group consisting of ectodermal, in particular neuroectodermal, neurogenic, mesenchymal or epithelial origin and metastases thereof, colon carcinomas, melanomas, squamous cell carcinomas, renal carcinomas, lymphomas, preferably Hodgkin lymphoma or non-Hodgkin lymphoma, e.g. diffuse large B-cell lymphoma (DLBCL), Ewing's sarcoma, lipsarcome, fibrosarcoma, leiomyosarcoma, meloma, thymoma, acute myeloid leukaemia (AML), acute lymphoid leukaemia (ALL), chronic myeloid leukaemia (CML), chronic lymphocytic leukaemia (CLL), gastrointestinal tumors, pulmonary carcinomas, gliomas, thyroid tumours, mammary carcinomas, prostate tumours, hepatomas, various virus-induced tumours such as, for example, papilloma virus-induced carcinomas (e.g. cervical carcinoma), adenocarcinomas, herpes virus-induced tumours (e.g. Burkitt's lymphoma, EBV-induced B-cell lymphoma), hepatitis B-induced tumours (hepatocell carcinoma), HTLV-1 and HTLV-2 induced lymphomas, acoustic neuromas/neurinomas, cervical cancer, lung cancer, pharyngeal cancer, anal carcinomas, neuroblastomas, gliomas, glioblastomas, lymphomas, rectal carcinomas, astrocytomas, brain tumours, stomach cancer, retinoblastomas, basaliomas, brain metastases, medulloblastomas, vaginal cancer, pancreatic carcinoma, testicular cancer, melanomas, thyroidal carcinomas, bladder cancer, Hodgkin's syndrome, meningiomas, Schneeberger disease, bronchial carcinomas, hypophysis tumour, Mycosis fungoides, oesophageal cancer, breast cancer, cardinoids, neurinomas, spinaliomas, laryngeal cancer, renal cancer, thymomas, corpus carcinomas, bone cancer, osteosarcomas, urethral cancer, CUP syndrome, head/neck tumours, oligodendrogliomas, vulval cancer, intestinal cancer, colon carcinomas, oesophageal carcinomas, tumors of the small intestine, craniopharyngeomas, ovarian carcinomas, gastric carcinoma, colonic carcinoma, soft tissue tumours/sarcomas, ovarian cancer, liver cancer, pancreatic carcinomas, cervical carcinomas, endometrial carcinomas, liver metastases, hepatocellular carcinoma, penile cancer, tongue cancer, gall bladder cancer, leukemia, plasmocytomas, uterine cancer, lid tumour, prostate cancer, malignant melanoma and blastomas. Particularly, solid tumours of ectodermal, in particular neuroectodermal, neurogenic, mesenchymal or epithelial origin and metastases thereof or non-solid tumors of the lymphatic or myeloid system may be addressed by the use according to the present invention.

The bispecific, in particular trifunctional, antibody or the pharmaceutical composition to be used according to the invention may be preferably serve for the treatment of solid or non-solid cancer diseases selected from the group consisting of gastric carcinoma, adenocarcinoma, melanoma, in particular malignant melanoma, glioma, colonic carcinoma, pancreatic carcinoma, ovarian carcinoma, uterine cancer, breast cancer, in particular metastatic breast cancer, hepatocellular carcinoma, bronchial carcinoma, leukemias, preferably acute or chronic leukemias of myeloid or lymphoid origin, lymphomas, preferably Hodgkin-lymphoma, non-Hodgkin lymphoma, e.g. diffuse large B-cell lymphoma (DLBCL), Ewing's sarcoma, liposarcome, fibrosarcoma, leiomyosarcoma, myeloma, thymoma and other soft tissue sarcomas and blastomas. More specifically, blastomas may be selected from the group consisting of hepatoblastoma, medulloblastoma, nephroblastoma, neuroblastoma, pancreatoblastoma, pleuropulmonary blastoma, retinoblastoma and glioblastoma.

The mammal to be treated is human or non-human animal. Amongst humans, the inventive treatment protocol by administering bispecific, in particular trifunctional, antibodies subcutaneously may be particularly useful for the treatment of any group of cancer patients. In particular, young (less than 15 year old) or elderly (more than 60 year old) cancer patients, in particular young and elderly patients suffering from non-solid tumors, e.g. lymphomas or leukemia, may be treated according to the present invention. Cancer patients, irrespective of their age, who are not under immunosuppressive treatment, may particularly benefit from the use of the bispecific, in particular trifunctional, antibody according to the invention.

### EXAMPLES

### Example 1

### Patient and treatment schedule:

In this pilot study, a Hodgkin-Lymphoma patient (41 years old, Hodgkin's disease, nodular sclerotic type, CD30, CD20, CD15 positive, diagnosed 14 years before the present study started) was chosen because the patient did not receive any previous immunosuppression in the last year before treatment, in order to avoid any impact of immunosuppression on the immunological effects of the trifunctional antibody, e.g. the cytokine release. The patient had various treatments before, like BMOP chemotherapy in change with cyclophosphamide and adriamycin with partial remission followed by mantle field radiation stopped after 2nd radiation due to severe side effects. Progressive disease was treated with ESHAP chemotherapy without remission. This was followed by a change of chemotherapy to gemcitabine/navelbine and dexamethasone with partial remission. Several chemotherapies were partly combined with radiotherapy (72Gy) mediastinal and both sides hilar. Full remission, then slight mediastinal recurrence and treatment with Mabthera for 8 months, whereby the Mabthera treatment was terminated 9 months before the present study started.until May 2013. Retroperitoneal positive lymph nodes (metastasis of M. Hodgkin) were diagnosed recently (a few days) before the present study started.

The patient received a cumulative dose of 2150 µg of the trifunctional antibody FBTA05/Lymphomun (anti-CD20 x anti-CD3) subcutaneously within 16 days. The trifunctional antibody FBTA05 (anti-CD20 x anti-CD3) was administered at escalating doses, whereby a single dose of 50 µg was administered at day 1, a single dose of 100 µg was administered at day 4, a single dose of 200 µg was administered at day 8, a single dose of 400 µg was administered at day 10, a single dose of 600 µg was administered at day 14 and a single dose of 800 µg was administered at day 16.

### Antibody tested, drug formulation and administration:

The trifunctional bispecific monoclonal antibody FBTA05 (also known as "Bi20" and "Lymphomun®") is a heterologous antibody, composed of two potent heavy chain subclasses, a mouse IgG2a and a rat IgG2b chain, each with their respective light chains. It therefore possesses two specific and one functional binding site. FBTA05 binds with one binding arm to the CD3 antigen on T cells and with the second binding arm to the CD20 antigen, which is expressed exclusively on normal and malignant B cells, but not on hematological precursor cells or other human cell types. In addition, the third functional site within the hybrid Fc region binds to Fcγ receptor type I, IIa and III, which are expressed by accessory cells (e.g., macrophages, dendritic cells, natural killer cells) of the immune system.

FBTA05/Lymphomun was supplied by TRION Research (Munich, Germany) as a sterile, pyrogen-free, color-free and preservative-free solution. The concentrate contained 0.1 mg/ml antibody in a 100mM sodium citrate buffer (pH 5.6), with 0.02% Tween 80. Depending on the dose level, Lymphomun was further diluted in 0.9 % sodium chloride solution for s.c. injection. The antibody was administered by s.c. injection into the abdominal wall, whereby per treatment ("dose") a total injection volume of 0.5 - 2 ml was administered within 1 - 5 min/injection.

### Clinical and experimental response evaluation:

Hematological and immunological parameters (for example, blood counts, FBTA05 levels, cytokine levels and lymphocyte subsets) were assessed in blood/serum/plasma samples of the patient obtained before and/or after each FBTA05 injection until day 19 after start of the therapy.

### Pharmakokinetiks / Quantification of FBTA05 in plasma:

FBTA05 concentrations in plasma samples were determined by ELISA. The test method is an enzyme-linked immunosorbent assay for the specific and selective detection and quantification of unbound FBTA05 antibody in human plasma samples. In the assay the rat/mouse hybrid antibody FBTA05 (analyte) is bound twice via an anti-rat specific capture antibody and an anti-mouse specific biotin-labeled detection antibody. The colorimetric reaction of the β-galactosidase substrate is measured at 570 nm using an ELISA plate reader. Reference standard dilutions are measured on each 96 well plate to generate a calibration curve. Samples are measured in duplicates. The lower limit of quantification (LLOQ) is 250 pg/mL. The assay was validated according to "Guidance for Industry: Bioanalytical MethodValidation" (CDER, May 2001).

### Measurement of cytokines:

Cytokines of plasma samples were measured applying multiplex Luminex technology. Briefly, plasma samples were collected at the study site, frozen (-20°C) and measured in batch using an 8-plex Fluorokine X-MAP kit (R&D Systems) which comprises the cytokines IL-2, IL-4, IL-6, IL-8, IL-10, IL-17, TNF-α and IFN-γ. Internally color coded microspheres with two fluorescent dyes and a specific antibody coated are used to detect above mentioned cytokines in the plasma sample. After an analyte is captured by a microsphere, a biotinylated detection antibody is introduced. The reaction mixture was then incubated with a Strepavidin - PE conjugate (reporter molecule) to complete the reaction on each microsphere. The microspheres were then measured in a Luminex® Xmap® system by passing rapidly through a laser which excited the internal dyes marking the microsphere set. A second laser excited the reporter molecule PE. Each individual microsphere was identified and the results were quantified based on fluorescent reporter signals on a Luminex 200 system. Cytokine determination is validated at TRION Research GmbH.

### Quantification of blood lymphocytes:

The following blood lymphocyte subpopulations were enumerated in percentage and absolute counts by flow cytometric analysis using the multitest IMK kit (Becton Dickinson, CE/IVD) and a FACS Calibur flow cytometer (Becton Dickinson, Heidelberg, Germany): mature CD3+ T cells, CD19+ B-cells, CD3+/CD4+ helper T-cells, CD8+/CD3+ T cells and CD3-, CD16+ CD56+ NK cells. The cells were quantified in counts / µl according to the manufacturer's instructions.

### Results:

### Response to treatment:

In general, the FBTA05 antibody injected subcutaneously was well tolerated Side effects like fever, chills and skin rash were reported. However, these side effects were considerably slighter and more transient as compared to the side effects upon i.v. administration, as reported for example by Buhmann et al. (2009) Bone Marrow Transplant, 43: 383-397. As a surrogate marker for a clinical/hematological response the B cells of the patient were measured before, during and after the treatment and depletion to zero could be demonstrated after the last s.c. injection (cf. Figure 3).

### Pharmacokinetic profile:

FBTA05 antibody concentrations in the plasma of the patient were assessed on each of days 1 to 5, 8 to 11, and 15 to 19 in order to obtain a pharmacokinetic profile of the trifunctional antibody FBTA05 (anti-CD20 x anti-CD3) after six subcutaneous administrations at single doses 50 µg, 100 µg, 200 µg, 400 µg, 600 µg and 800 µg (Figure 1). As shown in Fig. 1 antibody concentrations stayed in the range of about 2 ng/ml to 14 ng/ml from the second dose (100 µg at day 4) onwards until the end of the detection period at day 19, i.e. for more than two weeks. These results differ considerably from the pharmacokinetic profile of the trifunctional antibody FBTA05 administered via the intravenous route, where short lasting peak concentrations of about 10 - 380 ng/ml were detected for only 24 hours after administration (Buhmann R. et al. (2010) Haematologica 95 (supplement 2): p. 183)-).

### Cytokine release:

Figure 2 shows the concentrations of different cytokines, including proinflammatory cytokines IL-6, IL-8, IL-4 and TNFα and counter-inflammatory cytokines such as IL-10, in the plasma of the patient after subcutaneous administrations of FBTA05. Proinflammatory cytokines were detected generally in low amounts only, with a peak of up to 75 pg/ml for IL-6 on day 17, i.e. after administration the highest dose of 800 µg FBTA05. Inhibitory cytokines like IL-10, in contrast, were almost not detectable with concentrations remaining below 15 pg/ml over the treatment and detection period.

These results differ considerably from the cytokine release data upon FBTA05 administration in the prior art, which relate to i.v. administration. For example, Buhmann et al. investigated i.v. administration of escalating doses of FBTA05 in CLL (chronic lymphoid leukemia) and HG-NHL (high-grade non-Hodgkin's lymphoma) patients with different treatment schedules for each patient and reported concentrations of IL-6 of up to 580 pg/ml upon single closes of more than 500 pg FBTA05 in the CLL patients (Buhmann et al. (2009) Bone Marrow Transplant, 43: 383-397: Fig. 3a and 4a). Moreover, Buhmann et al. also reported significant IL-10 concentrations upon i.v. administration of FBTA05 with peaks of at least about 150 - 2400 pg IL-10/ml (Buhmann et al., Fig. 3b and 4b). This is in stark contrast to the IL-10 data of the present example, wherein IL-10 concentrations never exceeded 15 pg/ml (cf. Figure 2).

### B cell counting:

Figure 3 shows the effect of increasing dosing of FBTA05, subcutaneously administered, on the concentration of B-cells in the blood. B-cells were identified by CD19 ("CD19+ B-cells "). As clearly demonstrated in figure 3, the blood of the patient was completely depleted of the CD19+ B-cells by the end of the treatment with subcutaneously administered FBTA05 antibody. It is assumed that such a complete depletion of B-cells is achieved by a sufficiently high initial dose of FBTA05. Low initial doses of FBTA05, in contrast, are known to stimulate undesirable "HAMA" reactions ("human anti-mouse antibodies") (cf. Getts DR et al. (2010) mabs, 2(6): 682-694). In particular, partial depletion of the B-cell population due to a low initial dose of antibodies is assumed to promote "HAMA". Thus, a complete depletion of B-cells - as achieved in the present example - is desireable to avoid "HAMA".

### T cell and natural killer (NK) cell counting:

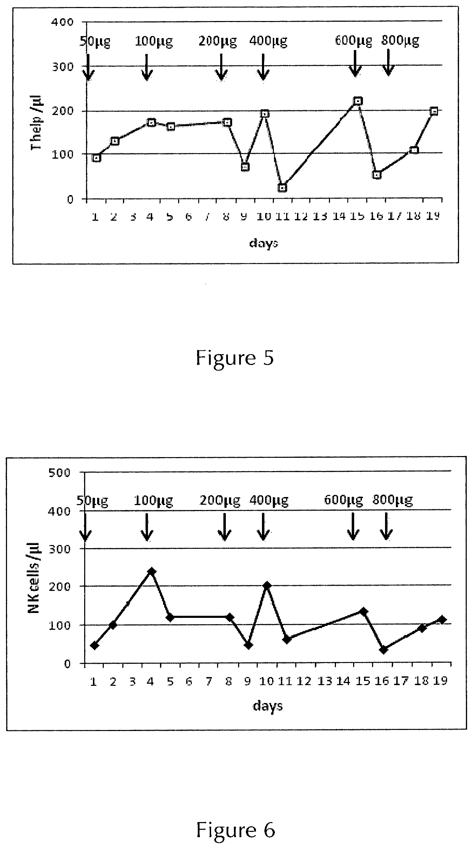

The T-cells play a central role in the cell-mediated immunity. To evaluate the in vivo efficacy of the subcutaneous administration of the trifunctional antibody FBTA05, the concentrations of CD8+ /CD3+ T-cells (Figure 4), CD4+ /CD3+ T-cells (T helper cells; Figure 5), as well as natural killer (NK) cells (Figure 6) were assessed over the course of FBTA05 treatment.

Figures 4 to 6 show the dose-effect relationship of subcutaneously administered trifunctional antibody FBTA05. Over the course of FBTA05 treatment, the concentration of T-cells and NK cells shows a dose-dependent modulation with a short peak and drop phase after each subcutaneous administration of the antibody.

Figure 4 shows the CD8+ /CD3+ T-cell count over the course of FBTA05 treatment, administered subcutaneously. An effect of transient lymphocytopenia occurs; i.e. a reduced absolute number of T cells in blood.

The higher the subcutaneous dosing of the trifunctional bispecific antibody FBTA05; i.e. 100 vs. 600 µg, the stronger is the effect observed on the concentration of CD8+/CD3+ T-cells; i.e. CD8+/CD3+ T-cell concentration decreasing to around 100 cells/µl after subcutaneous administration of 100 µg of the trifunctional bispecific antibody whereas the same value being almost zero after 600 µg of subcutaneous FBTA05 (cf. Figure 4).

A similar effect is also observed in Figures 5 and 6, which show the concentration of CD4+ /CD3+ helper T-cells over the course of FBTA05 treatment, administered subcutaneously, and the concentration of NK cells (CD16+, CD56+, CD3-) over the course of FBTA05 treatment, administered subcutaneously, respectively.

### General discussion of the results in view of the prior art:

The effects of the trifunctional bispecific antibody FBTA05 (anti-CD3 X anti-CD20) were investigated by Buhmann et al. upon intravenous administration (Buhmann et al. (2009) Bone Marrow Transplant, 43: 383-397). Six patients with CLL (Chronic Lymphoid Leukemia), BLL (Burkitt-like Lymphoma) and DLBCL (Diffuse Large B-Cell Lymphoma) were enrolled in the study and treated with intravenous FBTA05 (anti-CD3 X anti-CD20) with escalating doses.

The doses administered varied from 10 µg to 2000 µg. For instance, patient no. 1 with advanced CLL received a test dose of 10 µg on day 1, which was doubled every second day and increased up to 600 µg on day 17, followed by DLI (Donor Lymphocyte Infusion) (1 x 10⁷ CD3+ cells per kg of recipient body weight) on day 18. In patient no. 2 FBTA05 was escalated up to 1000 µg followed on day 28 by DLI (1 x 10⁶ CD3+ cells per kg of recipient body weight). DLI was further escalated up to 1 x 10⁷ CD3+ cells per kg on day 44 and 1 x 10⁸ CD3+ per kg on day 44 and 1 x 10⁸ CD3+ cells per kg on day 57, preceded by two applications of 1000 µg on days 52 and 56, respectively, followed by 500 µg on day 72 and 1000 µg on day 76. The antibody was administered as a continous 6-12 hour intravenous infusion by a precision infusion pump.

In all six patients inflammatory reactions such as fever and chills were observed. In CLL patients adverse effects appeared at dose levels of 40-160 µg.

More importantly, the increasing intravenous concentrations of FBTA05 favoured the release of IL-8 and especially IL-10. The measurement of the cytokine profile revealed in cases of CLL and HG-NHL a transient but significant increase of IL-6, IL-8 and IL-10 at increasing concentrations of FBTA05 accompanied by an increase of the C-reactive protein.

As it becomes evident from Example 1 of the present invention, an advantage of the present invention is based on higher cumulative doses per treatment cycle in comparison to prior art administration of trifunctional antibodies by the intravenous or intraperitoneal route.

Additionally, the present invention enables a faster increase in dosing in contrast to the intravenous treatment with trifunctional bispecific antibodies. As it can be seen in example 1 of the present invention, a starting dose of 50 µg was doubled to 100 µg, 200 µg, 400 µg, 600 µg every 2 days, whereas the intravenous administration of the same antibody, as described by the study of Buhmann et al, had to start at an initial low dose of only 10 µg due to the strong potential of trifunctional antibodies binding to the CD3 antigen to activate T cells triggering proinflammatory cytokines and upregulation of co-stimulatory molecules, e.g. CD28. Thus, the present invention achieves a shortened treatment cycle, which also results in a decreased injection frequency and therefore increases also the patient compliance as well as the convenience of the patient.

A further advantage of the present invention resides in the fact that subcutaneous administration of FBTA05 substantially suppresses the release of inhibitory cytokines, for instance, IL-10; see figure 2 of the present invention. Thus, a possible IL-10 mediated counteraction to FBTA05 mediated anti-tumor immunity can be avoided.

### Example 2:

### Patient and treatment schedule:

In this pilot study, a patient with metastatic breast cancer (female, age 54) was treated with escalating doses of the trifunctional antibody ertumaxomab (anti-HER2 x anti-CD3), administered subcutaneously. The treatment schedule included an initial dose of 50 µg ertumaxomab on day 1, followed by 100 µg ertumaxomab on day 4.

### Antibody tested, drug formulation and administration:

Ertumaxomab is a trifunctional bispecific monoclonal antibody targeting HER2/neu and CD3. It is usually produced by a quadroma cell line prepared by the fusion of a specific rat (anti-CD3) and mouse hybridoma cell line (anti-HER2). The heavy chain is composed of murine IgG2a and rat IgG2b subclasses with particularly selective binding to activatory Fcγ type I/III receptors. Together with its two antigen-binding sites, ertumaxomab is able to bind HER2/neupositive tumor cells, T cells, and simultaneously via its Fc portion, Fcγ receptor-positive accessory cells.

Ertumaxomab was supplied by TRION Research (Munich, Germany)/ manufactured according to (Lindhofer H et al. (1995) J Immunol;155(1): 219-25) as a sterile, clear, color-free and preservative-free concentrate. The concentrate contained 0.1 mg/ml antibody in a 100mM sodium citrate buffer. Depending on the dose level, Ertumaxomab was further diluted in 0.9 % sodium chloride solution for s.c. injection. The antibody was administered by s.c. injection into the abdominal wall, whereby per treatment ("dose") a total injection volume of 0.5 - 2 ml was administered within 1 - 5 min/injection. The patient experienced slight and transient skin rash, fever and chills. However, these side effects were considerably slighter and more transient as compared to the side effects upon i.v. administration, as reported for example by Kiewe et al. (2006) Clin Cancer Res, 12: 3085-3091.

### Clinical and experimental response evaluation:

Hematological and immunological parameters (for example, cytokine) were assessed in blood/serum samples of the patient obtained before and/or after each ertumaxomab injection

### Measurement of cytokines:

Measurement of cytokines was performed as described for Example 1.

### Results:

### Response to treatment:

In general, the ertumaxomab antibody injected subcutaneously was well tolerated, only slight and transient side effects like fever, chills and skin rash were observed. A hematological reaction could be also measured in the peripheral blood as T lymphocyte count was reduced from 1308 before s.c. injection of ertumaxomab to 652, 24h after the first treatment. Accordingly a reduction of CD8+/CD3+ T cells from 572 (before treatment) to 270 (after 24 h) and CD4+/CD3+T helper cells from 724 (before treatment) to 350 (after 24h) and NK cells from 220 (before treatment) to 177 (after 24h) was observed after the first injection.

### Cytokine release:

Table 1 shows the concentrations of different cytokines, including proinflammatory cytokines IL-6 and TNFα and counter-inflammatory cytokines such as IL-10, in the plasma of the patient after subcutaneous administration of ertumaxomab. Proinflammatory cytokines IL-6 and TNFα were detected in low amounts only, with peaks of up to 12 pg/ml for IL-6 after administration of an initial dose of 50 µg ertumaxomab. Inhibitory cytokines, like IL-10, were almost undetectable with concentrations remaining below 15 pg/ml.

**Table 1: The amount of cytokines measured in pg/mL as a result of 50 µg, and 100 µg subcutaneously administed ertumaxomab**

| Cytokine | 50µg s.c. | | | 100µg s.c. | |
|---|---|---|---|---|---|
| pg/ml | Day 1 | | | Day 4 | |
| | before | after | 24h after | before | after |
| INF gamma | < 3 | < 3 | < 3 | < 3 | < 3 |
| IL 10 | 12 | 12 | 13 | 12 | 12 |
| IL 17 | < 3 | < 3 | < 3 | < 3 | < 3 |
| IL 2 | < 3 | < 3 | < 3 | < 3 | < 3 |
| IL 4 | < 3 | < 3 | < 3 | < 3 | < 3 |
| IL 6 | < 3 | < 3 | 12 | < 3 | < 3 |
| TNF alpha | < 3 | < 3 | < 3 | < 3 | < 3 |

These results differ considerably from the cytokine release data upon ertumaxomab i.v. administration in the prior art. For example, Kiewe et al. investigated i.v. administration of ertumaxomab in patients with metastatic breast cancer with different treatment schedules reported concentrations of IL-6 with peak values of more than 1000 pg/ml and concentrations of IFN gamma and TNF alpha with peak values of more than 100 pg/ml (Kiewe et al. (2006) Clin Cancer Res, 12: 3085-3091; Fig. 3). In contrast, the results shown in Table 1 demonstrate that the trifunctional bispecific antibody ertumaxomab administered subcutaneously reduces proinflammatory cytokines in comparison to the intravenous route as reported by Kiewe et al.. General discussion of the results in view of the prior art:
Ertumaxomab (anti-Her2 x anti-CD3) was also tested on 17 patients with metastatic breast cancer, which were enrolled into a multicenter phase I dose-escalating trial. Three ascending doses of ertumaxomab (10-200 µg) were administered intravenously; i.e. 6 hour intravenous infusion, on day 1, 7 and 13. Safety and tolerability were the primary objectives. Secondary objectives were antitumor activity and different immunologic variables (Kiewe et al. (2006) Clin Cancer Res, 12: 3085-3091). Drug-related side effects (including fever, rigors, headache, nausea and vomiting) were observed. Kiewe et al mentioned that the safety data generated in said study implies that doses up to 100 µg could be infused safely. The individual patient, however, has to be closesly monitored. In contrast, the present invention clearly shows that much higher doses; i.e. 200 µg can be administered.

### Example 3

### Patient and treatment schedule:

In this study, a patient having recividated, Rituximab-refractory, CD20+ diffuse large B-cell lymphoma (DLBCL) with primary cutaneous manifestation was treated. At the beginning of the treatment, the number of CD20+ B-cells in the blood of the patient was in normal range (251/µl).

The patient received a cumulative dose of 6000 µg (6 mg) of the trifunctional antibody FBTA05/Lymphomun (anti-CD20 x anti-CD3) subcutaneously within 21 days (7 injections in total). The trifunctional antibody FBTA05 (anti-CD20 x anti-CD3) was administered at escalating doses, whereby a single dose of 100 µg was administered at day 0, a single dose of 200 µg was administered at day 3, a single dose of 400 µg was administered at day 7, a single dose of 800 µg was administered at day 10, a single dose of 1000 µg was administered at day 14, a single dose of 1500 µg was administered at day 17 and a single dose of 2000 µg was administered at day 21, as shown below in Table 2.

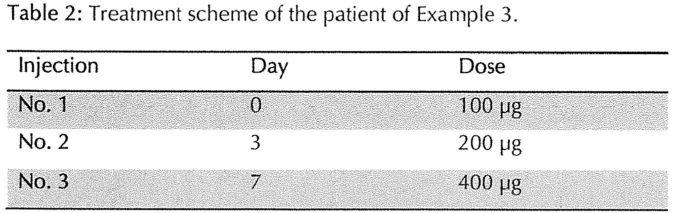

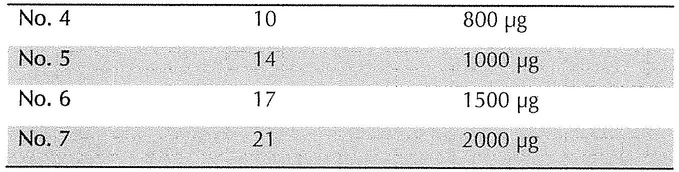

### Antibody tested, drug formulation and administration:

As described above, in Example 1, the trifunctional bispecific monoclonal antibody FBTA05 (also known as "Bi20" and "Lymphomun®") is a heterologous antibody, composed of two potent heavy chain subclasses, a mouse IgG2a and a rat IgG2b chain, each with their respective light chains. It therefore possesses two specific and one functional binding site. FBTA05 binds with one binding arm to the CD3 antigen on T cells and with the second binding arm to the CD20 antigen, which is expressed exclusively on normal and malignant B cells, but not on hematological precursor cells or other human cell types. In addition, the third functional site within the hybrid Fc region binds to Fcγ receptor type I, IIa and III, which are expressed by accessory cells (e.g., macrophages, dendritic cells, natural killer cells) of the immune system.

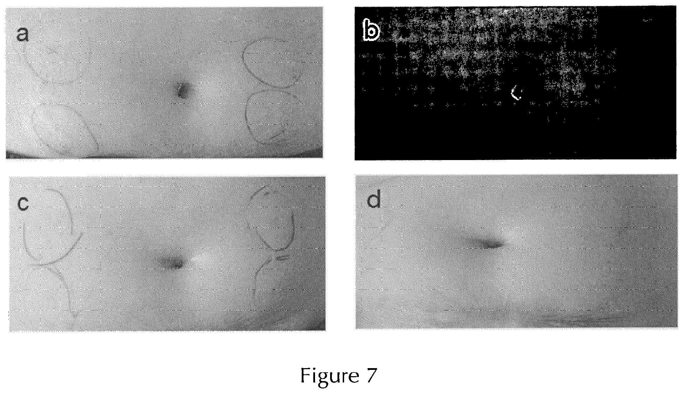

FBTA05/Lymphomun was supplied by TRION Research (Munich, Germany) as a sterile, pyrogen-free, color-free and preservative-free solution. The concentrate contained 1,1 mg/ml antibody in a 20mM sodium citrate buffer (pH 6.0). Depending on the dose level, Lymphomun was further diluted in 0.9 % sodium chloride solution for s.c. injection. The antibody was administered by s.c. injection into the abdominal wall, whereby on every treatment day four single injections into different sites around the navel were performed. Thus, the total volume per treatment day, which was 2000 µl, was distributed in four single injections of 500 µl each. In other words, on every treatment day, the dose (see Table 2) was evenly distributed on four injections of 500 µl each. A single injection was performed within 1 - 5 min/injection. Antibody administration was well tolerated. Moderate redness of the skin at the injection sites were sufficiently treated by topical application of cortisone (cream; cf. Figure 7).

### Clinical and experimental response evaluation:

Hematological and immunological parameters (for example, blood counts, FBTA05 levels, cytokine levels and lymphocyte subsets) were assessed in blood/serum/plasma samples of the patient obtained before and/or after each FBTA05 injection until day 34 after start of the therapy. Measurements (pharmacokinetics, quantification of FBTA05 in plasma, measurement of cytokines, quantification of blood cells) were performed as described in Example 1.

### Results:

### Response to treatment:

In general, the FBTA05 antibody injected subcutaneously was very well tolerated on local as well as on systemical level. Thus, it was sufficient to monitor the patient as an inpatient on the treatment day for 24 h. Thereafter, the patient could leave the hospital until the next treatment. Slight side effects like transient local skin reactions at the injection sites, periorbital edema on day 10 with regression on day 14, and increased restlessness and insomnia were reported. However, these side effects were considerably slighter and more transient as compared to the side effects upon i.v. administration, as reported for example by Buhmann et al. (2009) Bone Marrow Transplant, 43: 383-397.

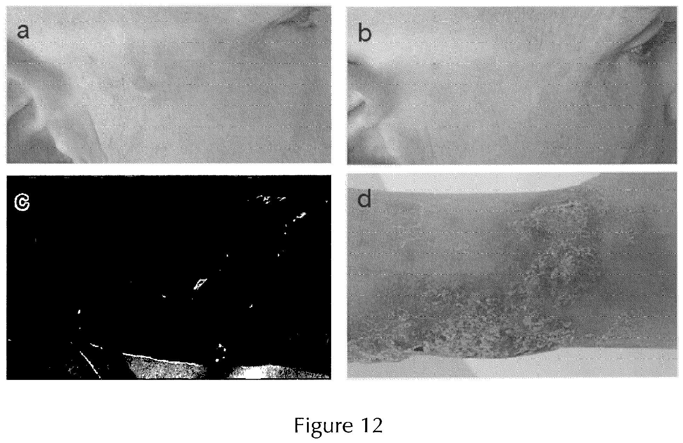

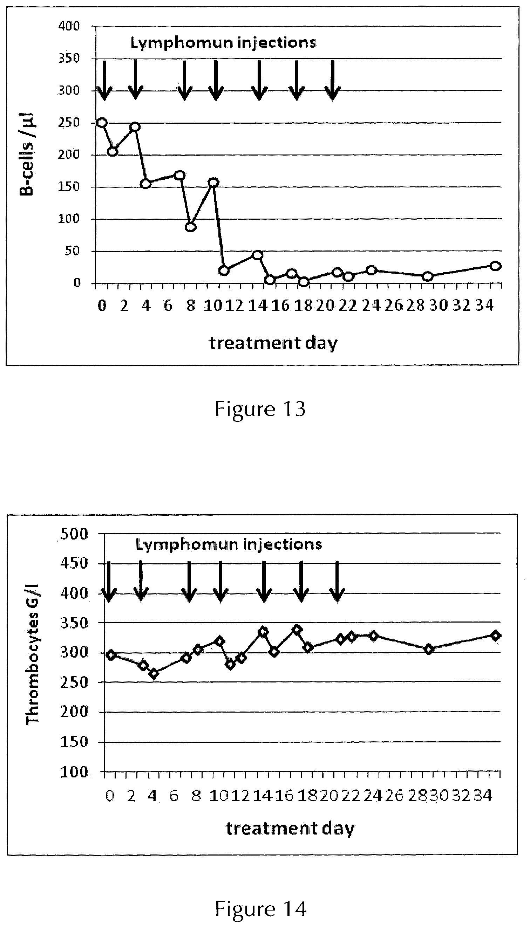

As a surrogate marker for a clinical/hematological response the cutaneous tumor regression was observed and the B cells of the patient were measured before, during and after the treatment and depletion to zero could be demonstrated after the last s.c. injection (cf. Figures 12 and 13).

### Pharmacokinetic profile:

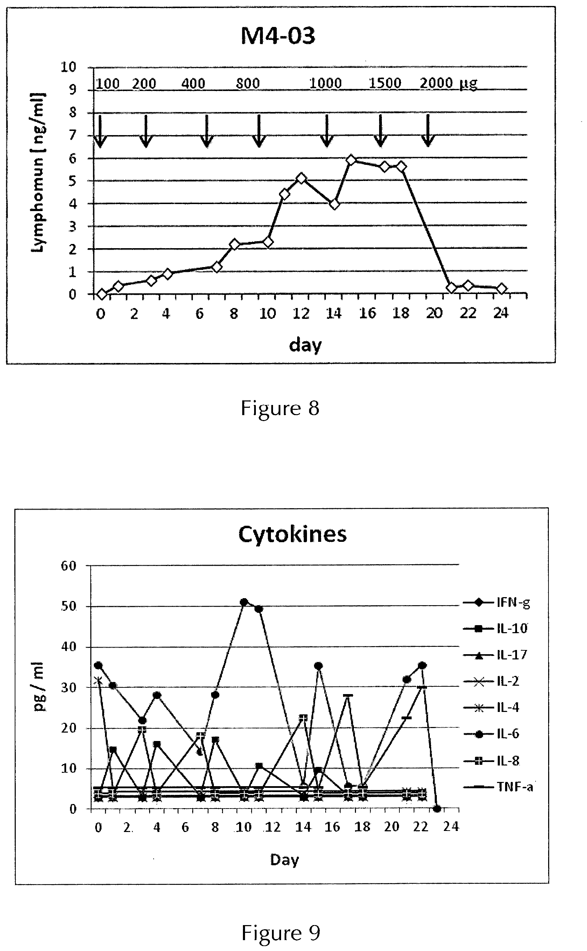

FBTA05 antibody concentrations in the plasma of the patient were assessed on each of days 0, 1, 3, 4, 7, 8, 10, 11, 12, 14, 15, 17, 18, 21, 22 and 24 in order to obtain a pharmacokinetic profile of the trifunctional antibody FBTA05 (anti-CD20 x anti-CD3) after seven subcutaneous administrations at single doses 100 µg, 200 µg, 400 µg, 800 µg, 1000 µg, 1500 µg and 2000 µg (Figure 8). As shown in Fig. 8 maximal concentrations of the antibody were 5 - 6 ng/ml. According to in vitro studies Lymphomun concentrations of 5 ng/ml and above are maximally effective and, thus, a strong antitumor activity was shown at such concentrations. Thus, in the present treatment scheme effective concentrations of the therapeutic antibody were achieved in particular from a dose of 800 µg. When compared to intravenous administration no peaks in concentration immediately after the injection were observed (Buhmann et al. (2009) Bone Marrow Transplant, 43: 383-397; Schuster et al. (2015) Br. J Haematol. 169 (1): 90-102). Instead, after subcutaneous administration concentration of the antibody in the blood increases slower, so that only 1 - 2 days after administration the maximal concentration is reached. Furthermore, also the decrease in antibody concentration takes several days and is thus considerably slower compared to intravenous administration. The observed plateau in concentrations points to a depot effect in the skin.

### Cytokine release:

Figure 9 shows the concentrations of different cytokines, including proinflammatory cytokines IL-6, IL-8 and TNFα and counter-inflammatory cytokines such as IL-10, in the plasma of the patient after subcutaneous administrations of FBTA05 (Lymphomun). Proinflammatory cytokines were detected generally in low amounts only, with the most prominent detectable proinflammatory cytokine being IL-6 showing a peak of up to 50 pg/ml. In addition, also IL-8 and TNFα could be detected. In addition, a weak counterreaction with a slight increase in IL-10 was observed. IL-17, IL-2, IFN-g and IL-4 were not detectable.

As discussed in detail in the context of Example 1, these results differ considerably from the cytokine release data upon FBTA05 administration in the prior art, which relate to i.v. administration.

### CRP measurement:

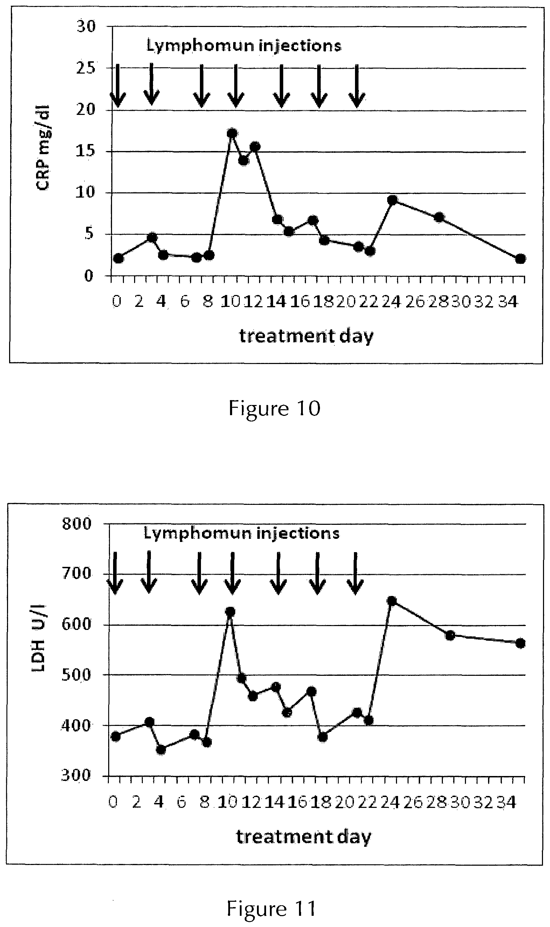

C-reactive protein (CRP) represents an important inflammatory parameter, and proinflammatory IL-6 is known to stimulate CRP synthesis. In the present case, the patient showed already before the start of the antibody therapy a slightly increased CRP-value of 2.2 mg/dl, which further increased after the third antibody injection to 17.2 mg/dl (Figure 10). That increase coincides with the increase in IL-6 (Figure 9) and indicates the immunological effectiveness of the antibody therapy. In course of the further treatment CRP blood levels decreased and after 14 days after the last administration CRP levels returned to the base level.

### LDH measurement:

Increased bloodlevels of the metabolic enzyme lactate dehydrogenase (LDH) indicate an increased cell death. In oncology, increased LDH values can be attributed to progression of a tumor as well as to increased lysis of a tumor, whereby both processes may also overlap. In the present Example the LDH values show a transient increase following the third antibody treatment (400 µg dose) as well as following the seventh antibody treatment (dose: 2000 µg; Figure 11). The transient LDH increase following the third antibody administration may point to a beginning lysis of the tumor. This is supported by the strong immunological reaction with increased IL-6 and CRP values.

### Clinical evaluation:

In the clinical evaluation antibody treatment results in an impressive regression of the tumor (Figure 12). The cutaneous tumor lesion of the right side of the face (Fig. 12a, b) regressed completely, the lesions with manifestation at the right lower leg are in regression (Fig. 12c, d). The cutaneous lesions, which were initially infiltrating and extending into the surrounding healthy tissue (Fig. 12c), are now clearly confined against the healthy skin regions and their prominence as well as pain upon touch are decreased. In addition, the treatment resulted in a considerable improvement of the subjective well-being, objectified by an increase in body weight upon increased appetite as well as an improved mobility at decreased pain symptomatology.

### B cell counting:

Figure 13 shows the effect of increasing dosing of FBTA05 (Lymphomun), subcutaneously administered, on the concentration of B-cells in the blood. B-cells were identified by CD19 ("CD19+ B-cells "). During the antibody therapy a complete and sustained depletion of B-cells in the blood was achieved. Already after the fourth antibody dose (800 µg), B-cells were reduced to 20/µl from an initial value of of 251/µl. In the following course of treatment the B-cells remained at that low level. As clearly demonstrated in Fig. 13, the blood of the patient was completely depleted of the CD19+ B-cells by the end of the treatment with subcutaneously administered Lymphomun antibody. Interestingly, the low number of peripheral B cells was still observed even 14 days following the last antibody injection (Fig. 13). The sustained elimination of CD19+ B-cells in the blood indicates the effectiveness of the therapy due to an induced and sustained immune response.

### Thrombocyte, T-cell and natural killer (NK) cell counting:

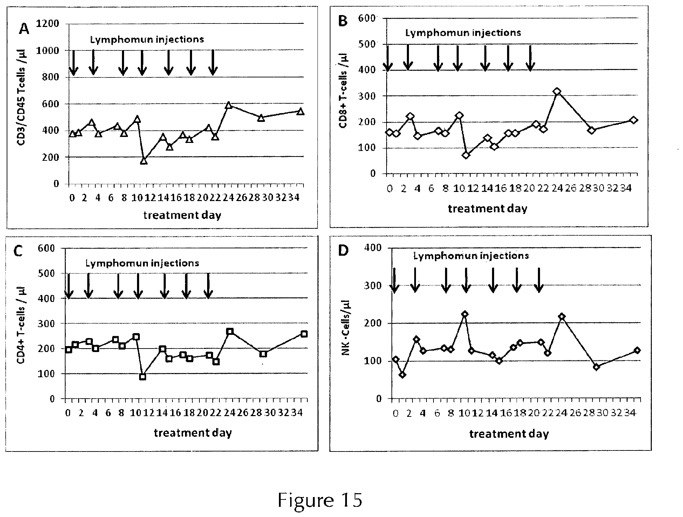

The T-cells play a central role in the cell-mediated immunity. To evaluate the in vivo efficacy of the subcutaneous administration of the trifunctional antibody FBTA05, the concentrations of thrombocytes (Figure 14) as well as the concentrations of CD3+/CD45+ T-cells (Figure 15A), CD8+ T-cells (Figure 15 B), CD4+ T-cells (Figure 15 C), and CD16+/CD56+ natural killer (NK) cells (Figure 15D) were assessed over the course of FBTA05 (Lymphomun) treatment.

Figure 14 shows that the number of thrombocytes remained stable over the course of treatment and was almost continuously in the normal range.

The immunotherapy exerted a positive effect on the number of T-cells, which increasd from 378/µl at the beginning to more than 500/µl (Figure 15 A). CD4+ as well as CD8+ T-cells contributed to that increase in T-cells. In contrast to previous observations upon intravenous administration of the antibody (Buhmann et al. (2009) Bone Marrow Transplant, 43: 383-397; Schuster et al. (2015) Br. J Haematol. 169 (1): 90-102), only slight fluctuations in the number of T-cells and NK cells (Figure 15B-D) as well as in the number of thrombocytes (Figure 14) were observed following subcutaneous administration of the antibody.

### Advantages of the present invention

The present invention relates to the discovery that a bispecific, in particular trifunctional, antibody can be administered to patients suffering from a cancer disease as an immune therapy, optionally as a concomitant therapy in combination with another anti-cancer agent without evoking adverse side effects observed according to prior art treatment protocols.

The treatment protocol according to the present invention, which includes administration of repeating doses of the antibody as defined herein subcutaneously, reduces the (exceeding) release of proinflammatory cytokines and, simultaneously, suppresses the release of inhibitory cytokines (e.g. IL-10) in a mammal to be treated. Cytokine release as described above was observed in the art upon intravenous or intraperitoneal administration of bispecific, in particular trifunctional, antibodies, e.g. by applying triple infusions of ertumaxomab (anti-HER2 X anti-CD3) intravenously. While such a treatment regimen yields a strong immunologic response (Kiewe et al. (2006) Clin Cancer Res, 12: 3085-3091), the adverse effects (nausea, fever, headache) and the dampening effects exerted by inhibitory cytokines on the immune system are avoided by the inventive use of bispecific, in particular trifunctional, antibodies.

It was based on an unexpected finding that subcutaneous administration does not evoke such adverse effects or, if at all, only to a significantly reduced extent. As a consequence of subcutaneous administration, cancer therapy by bispecific, in particular trifunctional, antibodies may be applied without frequent hospitalization avoiding exceedingly high costs. Additionally, the inventive finding paves the way for self-medication without requiring the assistance of a physician, thereby increasing the patient's compliance. That holds even more so, as subcutaneous administration of the bispecific, in particular trifunctional, antibody is done within a period of 5 minutes per injection, in particular within 1-5 minutes per injection, whereas administration by the intravenous route (Buhmann et al. (2009) Bone Marrow Transplant, 43: 383-397) requires infusion for 6-12 hours. Still, by subcutaneous administration of the bispecific, in particular trifunctional, antibody according to the present invention, direct destruction of the tumor cells and induction of immunity against the target tumor are observed. Thus, the present invention achieves an improvement in the anti-tumor activity by enhancing the immunologic effector functions of the bispecific, in particular trifunctional, antibody without evoking adverse effects.

Monoclonal monospecific antibodies do not exhibit any such T cell recruiting effects and do not trigger a comparable cytokine immune response, let alone a cytokine release profile as observed for bispecific, in particular trifunctional, antibodies upon intravenous administration. Accordingly, cross-reference to monoclonal monospecific antibodies does not contribute to the present invention, as distinct mechanisms of the immune system are addressed by these distinct types of antibodies. In any case, it may be worth mentioning that subcutaneous administration of monoclonal monospecific antibodies was considered to be promising at all. E.g., subcutaneous administration of Trastuzumab; i.e. a monoclonal monospecific antibody directed against the human epidermal growth factor receptor 2 (HER2), was studied. For a significant number of patients serious adverse events in the subcutaneous administration group (21%) were observed, even more than for the intravenous treatment group (12%). This difference was interpreted to be due to more infections at the injection site in the subcutaneous administration group than in the i.v. treatment group (8.1% vs. 4.4%) (Hamizi et al. (2013) OncoTargets and Therapy; 6: 89-94).

## Claims

1. Bispecific antibody for use in the treatment of a cancer disease, wherein said antibody is administered via the subcutaneous route, wherein the antibody is a T cell redirecting antibody and comprises a specificity against a T cell surface antigen, a specificity against a tumor-associated antigen and an Fc region.

2. The bispecific antibody for use according to claim 1, **characterized in that** the antibody has an IgG-like format.

3. The bispecific antibody for use according to claim 1 or 2, wherein the tumor-associated antigenis selected from the group consisting of EpCAM, HER2/neu, HER3/neu, G250, CEA, MAGE, proteoglycan, VEGF, EGFR, .alpha.V.beta.3-integrin, HLA, HLA-DR, ASC, CD1, CD2, CD4, CD6, CD7, CD8, CD11, CD13, CD14, CD19, CD20, CD21, CD22, CD23, CD24, CD30 CD33, CD37, CD40, CD41, CD47, CD52, c-erb-2, CALLA, MHCII, CD44v3, CD44v6, p97, ganglioside GM1, GM2, GM3, GD1a, GD1b, GD2, GD3, GT1b, GT3,GQ1, NY-ESO-1, NFX2, SSX2, SSX4 Trp2, gp100, tyrosinase, Muc-1, telomerase, survivin, G250, p53, CA125 MUC, Wue antigen, Lewis Y antigen, HSP-27, HSP-70, HSP-72, HSP-90, Pgp, MCSP, EpHA2 and cell surface targets GC182, GT468 or GT512 or from the group consisting of EpCAM, HER2/neu, HER3/neu, G250, CEA, MAGE, proteoglycan, VEGF, EGFR, .alpha.V.beta.3-integrin, HLA, HLA-DR, ASC, CD1, CD2, CD4, CD6, CD7, CD8, CD11, CD13, CD14, CD19, CD20, CD21, CD22, CD23, CD24, CD30 CD33, CD37, CD40, CD41, CD47, CD52, c-erb-2, CALLA, MHCII, CD44v3, CD44v6, p97, ganglioside GM1, GM2, GM3, GD1a, GD1b, GD2, GD3, GT1b, GT3,GQ1, NY-ESO-1, NFX2, SSX2, SSX4 Trp2, gp100, tyrosinase, Muc-1, telomerase, survivin, G250, p53, CA125 MUC, Wue antigen, Lewis Y antigen, HSP-27, HSP-70, HSP-72, HSP-90, Pgp, MCSP, EpHA2 and cell surface targets GC182, GT468, PD-L1, arboviral E protein or GT512.

4. The bispecific antibody for use according to any of claims 1 to 3, wherein the T cell surface antigen is selected from the group consisting of CD3, CD2, CD4, CD5, CD6, CD8, CD28, CD40L and/or CD44, preferably CD3.

5. The bispecific antibody for use according to any of claims 1 to 4, wherein said antibody has
(i) a first specificity against T cell surface antigen CD3 and a second specificity against a tumor-associated antigen selected from the group consisting of ganglioside GM1, GM2, GM3, GD1a, GD1b, GD2, GD3, GT1b, GT3 and GQ1 or selected from the group consisting of tumor antigen EpCAM, HER2/neu and CD20;
(ii) a first specificity against CD3 and a second specificity against the tumor-associated antigen GD2 or tumor antigen CD20 or tumor antigen EpCAM or the tumor-associated antigen HER2, preferably wherein said antibody is catumaxomab, FBTA05, Ektomun, or ertumaxomab; or
(iii) a first specificity against T cell surface antigen CD3 and a second specificity against a tumor-associated antigen selected from the group consisting of GD3, CD19, CD30, CD33, PD-L1, EGF-R and arboviral E protein.

6. The bispecific antibody for use according to any of claims 1 to 5 wherein said cancer disease is
(a) a solid or a non-solid tumor disease, preferably selected from the group consisting of gastric carcinoma, adenocarcinoma, melanoma, in particular malignant melanoma, colonic carcinoma, pancreatic carcinoma, ovarian carcinoma, uterine cancer, breast cancer, hepatocellular carcinoma, bronchial carcinoma, leukemias, preferably acute or chronic leukemias, lymphomas, preferably Hodgkin-lymphoma, Ewing's sarcoma, liposarcome, fibrosarcoma, leiomyosarcoma, myeloma, thymoma and other soft tissue sarcomas and blastomas, in particular neuroblastoma and glioma; or
(b) a Hodgkin-lymphoma or a non-Hodgkin-lymphoma, e.g. diffuse large B-cell lymphoma.

7. The bispecific antibody for use according to any of claims 1 to 6, wherein said antibody is administered to a patient diagnosed with at least one type of cancer wherein the treatment comprises
a. subcutaneously administering an initial dose of said antibody; and consecutively
b. subcutaneously administering at least one repeating dose of said antibody; wherein said at least one repeating dose is preferably unaltered as compared to the amount of the previous dose or greater than the amount of the previous dose, preferably wherein the at least one repeating dose exceeds the amount administered as previous dose by a factor of 1.5 to 5, more preferably by a factor of 1.5 to 3, even more preferably by a factor of 1.5 to 2.5.

8. The bispecific antibody for use according to any of claims 1 to 7, wherein said antibody is administered in combination with
(i) an anti-cancer drug or an anti-cancer antibody, preferably by separate administration; and/or
(ii) a glucocorticoid, wherein the glucocorticoid is preferably administered topically at the site of antibody administration.

9. A pharmaceutical composition for use in the treatment of a cancer disease, wherein said pharmaceutical composition comprises a bispecific, in particular a T cell redirecting, trifunctional, antibody as defined in any one of claims 1 - 8, wherein said composition is administered subcutaneously.

10. The pharmaceutical composition for use in the treatment of a cancer disease according to claim 9, wherein the pharmaceutical composition comprises a buffer, preferably an organic acid buffer, more preferably an organic acid buffer selected from the group consisting of citrate buffer, phosphate buffer, succinate buffer, tartrate buffer, even more preferably a citrate buffer, wherein the concentration of the buffer, preferably of the organic acid buffer, more preferably of the citrate buffer, is preferably no more than 100 mM, more preferably no more than 50 mM, even more preferably no more than 40 mM and particularly preferably no more than 30 mM, e.g. 20 mM.

11. Combination therapy for use in the treatment of a cancer disease, wherein the bispecific antibody according to any of claims 1 to 8 or the pharmaceutical composition according to claim 9 or 10 are administered subcutaneously in combination with at least one further anti-cancer drug or (monoclonal, monospecific) anti-cancer antibody, preferably administered separately from the bispecific antibody.

12. Combination therapy for use according to claim 11, wherein the at least one further anti-cancer antibody is selected from the group consisting of trastuzumab, alemtuzumab, bevacizumab, brentuximab vedotin, cetuximab, gemtuzumab ozogamicin, ibritumomab tiuxetan, ipilmumab, nimotuzumab, ofatumumab, panitumumab, rituximab and tositumomab or wherein the at least one further anti-cancer antibody is selected from the group consisting of nivolumab, pembrolizumab, and blinatumomab.

13. Combination therapy for use in the treatment of a cancer disease, wherein the bispecific antibody according to any of claims 1 to 8 or the pharmaceutical composition according to claim 9 or 10 are administered subcutaneously in combination with a glucocorticoid, wherein the glucocorticoid is preferably administered topically at the site of antibody administration.

## Patentansprüche

1. Bispezifischer Antikörper zur Verwendung bei der Behandlung einer Krebserkrankung, wobei der Antikörper über den subkutanen Weg verabreicht wird, wobei der Antikörper ein T-Zell-umlenkender Antikörper ist und eine Spezifität gegen ein T-Zell-Oberflächenantigen, eine Spezifität gegen ein Tumor-assoziiertes Antigen und eine Fc-Region umfasst.

2. Bispezifischer Antikörper zur Verwendung nach Anspruch 1, **dadurch gekennzeichnet, dass** der Antikörper ein IgG-ähnliches Format aufweist.

3. Bispezifischer Antikörper zur Verwendung nach Anspruch 1 oder 2, wobei das Tumor-assoziierte Antigen ausgewählt ist aus der Gruppe bestehend aus EpCAM, HER2/neu, HER3/neu, G250, CEA, MAGE, Proteoglykan, VEGF, EGFR, .alpha.V.beta.3-Integrin, HLA, HLA-DR, ASC, CD1, CD2, CD4, CD6, CD7, CD8, CD11, CD13, CD14, CD19, CD20, CD21, CD22, CD23, CD24, CD30 CD33, CD37, CD40, CD41, CD47, CD52, c-erb-2, CALLA, MHCII, CD44v3, CD44v6, p97, Gangliosid GM1, GM2, GM3, GD1a, GD1b, GD2, GD3, GT1b, GT3,GQ1, NY-ESO-1, NFX2, SSX2, SSX4 Trp2, gp100, Tyrosinase, Muc-1, Telomerase, Survivin, G250, p53, CA125 MUC, Wue-Antigen, Lewis-Y-Antigen, HSP-27, HSP-70, HSP-72, HSP-90, Pgp, MCSP, EpHA2 und Zelloberflächen-Targets GC182, GT468 oder GT512 oder aus der Gruppe bestehend aus EpCAM, HER2/neu, HER3/neu, G250, CEA, MAGE, Proteoglykan, VEGF, EGFR, .alpha.V.beta.3-Integrin, HLA, HLA-DR, ASC, CD1, CD2, CD4, CD6, CD7, CD8, CD11, CD13, CD14, CD19, CD20, CD21, CD22, CD23, CD24, CD30 CD33, CD37, CD40, CD41, CD47, CD52, c-erb-2, CALLA, MHCII, CD44v3, CD44v6, p97, Gangliosid GM1, GM2, GM3, GD1a, GD1b, GD2, GD3, GT1b, GT3,GQ1, NY-ESO-1, NFX2, SSX2, SSX4 Trp2, gp100, Tyrosinase, Muc-1, Telomerase, Survivin, G250, p53, CA125 MUC, Wue-Antigen, Lewis-Y-Antigen, HSP-27, HSP-70, HSP-72, HSP-90, Pgp, MCSP, EpHA2 und Zelloberflächen-Targets GC182, GT468, PD-L1, arbovirales E-Protein oder GT512.

4. Bispezifischer Antikörper zur Verwendung nach einem der Ansprüche 1 bis 3, wobei das T-Zelloberflächenantigen ausgewählt ist aus der Gruppe bestehend aus CD3, CD2, CD4, CD5, CD6, CD8, CD28, CD40L und/oder CD44, vorzugsweise CD3.

5. Bispezifischer Antikörper zur Verwendung nach einem der Ansprüche 1 bis 4, wobei der Antikörper
(i) eine erste Spezifität gegen das T-Zell-Oberflächenantigen CD3 und eine zweite Spezifität gegen ein Tumor-assoziiertes Antigen, ausgewählt aus der Gruppe bestehend aus Gangliosid GM1, GM2, GM3, GD1a, GD1b, GD2, GD3, GT1b, GT3 und GQ1 oder ausgewählt aus der Gruppe bestehend aus Tumorantigen EpCAM, HER2/neu und CD20, aufweist;
(ii) eine erste Spezifität gegen CD3 und eine zweite Spezifität gegen das Tumor-assoziierte Antigen GD2 oder das Tumorantigen CD20 oder das Tumorantigen EpCAM oder das Tumor-assoziierte HER2 aufweist, vorzugsweise wobei der Antikörper Catumaxomab, FBTA05, Ektomun oder Ertumaxomab ist; oder
(iii) eine erste Spezifität gegen das T-Zell-Oberflächenantigen CD3 und eine zweite Spezifität gegen ein Tumor-assoziiertes Antigen, ausgewählt aus der Gruppe bestehend aus GD3, CD19, CD30, CD33, PD-L1, EGF-R und arboviralem E-Protein aufweist.

6. Bispezifische Antikörper zur Verwendung nach einem der Ansprüche 1 bis 5, wobei die Krebserkrankung
(a) eine solide oder nicht solide Tumorerkrankung ist, vorzugsweise ausgewählt aus der Gruppe bestehend aus Magenkarzinom, Adenokarzinom, Melanom, insbesondere malignes Melanom, Kolonkarzinom, Pankreaskarzinom, Ovarialkarzinom, Gebärmutterkrebs, Brustkrebs, hepatozelluläres Karzinom, Bronchialkarzinom, Leukämien, vorzugsweise akute oder chronische Leukämien, Lymphome, vorzugsweise Hodgkin-Lymphom, Ewing-Sarkom, Liposarkom, Fibrosarkom, Leiomyosarkom, Myelom, Thymom und andere Weichteilsarkome und Blastome, insbesondere Neuroblastom und Gliom; oder
(b) ein Hodgkin-Lymphom oder ein Non-Hodgkin-Lymphom, z.B. ein diffuses großzelliges B-Zell-Lymphom ist.

7. Bispezifischer Antikörper zur Verwendung nach einem der Ansprüche 1 bis 6, wobei der Antikörper einem Patienten verabreicht wird, bei dem mindestens eine Krebsart diagnostiziert wurde, wobei die Behandlung umfasst
a. subkutane Verabreichung einer Initialdosis des Antikörpers; und anschließend
b. subkutane Verabreichung mindestens einer Wiederholungs-Dosis des Antikörpers; wobei die mindestens eine Wiederholungs-Dosis im Vergleich zu der Menge der vorherigen Dosis vorzugsweise unverändert oder größer als die Menge der vorherigen Dosis ist, vorzugsweise wobei die mindestens eine Wiederholungs-Dosis die als vorherige Dosis verabreichte Menge um einen Faktor von 1,5 bis 5, mehr bevorzugt um einen Faktor von 1,5 bis 3, noch mehr bevorzugt um einen Faktor von 1,5 bis 2,5 übersteigt.

8. Bispezifischer Antikörper zur Verwendung nach einem der Ansprüche 1 bis 7, wobei der Antikörper verabreicht wird in Kombination mit
(i) einem Anti-Krebs-Medikament oder einem Anti-Krebs-Antikörper, vorzugsweise durch getrennte Verabreichung; und/oder
(ii) einem Glucocorticoid, wobei das Glucocorticoid vorzugsweise topisch am Ort der Antikörperverabreichung verabreicht wird.

9. Pharmazeutische Zusammensetzung zur Verwendung bei der Behandlung einer Krebserkrankung, wobei die pharmazeutische Zusammensetzung einen bispezifischen, insbesondere einen T-Zell-umlenkenden, trifunktionalen Antikörper, wie in einem der Ansprüche 1 bis 8 definiert, umfasst, wobei die Zusammensetzung subkutan verabreicht wird.

10. Pharmazeutische Zusammensetzung zur Verwendung bei der Behandlung einer Krebserkrankung nach Anspruch 9, wobei die pharmazeutische Zusammensetzung einen Puffer, vorzugsweise einen organischen Säurepuffer, mehr bevorzugt einen organischen Säurepuffer ausgewählt aus der Gruppe bestehend aus Citratpuffer, Phosphatpuffer, Succinatpuffer, Tartratpuffer, noch mehr bevorzugt einen Citratpuffer, umfasst, wobei die Konzentration des Puffers, vorzugsweise des organischen Säurepuffers, mehr bevorzugt des Citratpuffers, vorzugsweise nicht mehr als 100 mM, mehr bevorzugt nicht mehr als 50 mM, noch mehr bevorzugt nicht mehr als 40 mM und besonders bevorzugt nicht mehr als 30 mM beträgt, z. B. 20 mM.

11. Kombinationstherapie zur Verwendung bei der Behandlung einer Krebserkrankung, wobei der bispezifische Antikörper nach einem der Ansprüche 1 bis 8 oder die pharmazeutische Zusammensetzung nach Anspruch 9 oder 10 subkutan in Kombination mit mindestens einem weiteren Anti-Krebs-Medikament oder einem (monoklonalen, monospezifischen) Anti-Krebs-Antikörper, vorzugsweise getrennt von dem bispezifischen Antikörper, verabreicht wird.

12. Kombinationstherapie zur Verwendung nach Anspruch 11, wobei der mindestens eine weitere Anti-Krebs-Antikörper ausgewählt ist aus der Gruppe bestehend aus Trastuzumab, Alemtuzumab, Bevacizumab, Brentuximab-Vedotin, Cetuximab, Gemtuzumab-Ozogamicin, Ibritumomab-Tiuxetan, Ipilimumab, Nimotuzumab, Ofatumumab, Panitumumab, Rituximab und Tositumomab, oder wobei der mindestens eine weitere Anti-Krebs-Antikörper ausgewählt ist aus der Gruppe bestehend aus Nivolumab, Pembrolizumab und Blinatumomab.

13. Kombinationstherapie zur Verwendung bei der Behandlung einer Krebserkrankung, wobei der bispezifische Antikörper nach einem der Ansprüche 1 bis 8 oder die pharmazeutische Zusammensetzung nach Anspruch 9 oder 10 subkutan in Kombination mit einem Glucocorticoid verabreicht wird, vorzugsweise wobei das Glucocorticoid topisch am Ort der Antikörperverabreichung verabreicht wird.

## Revendications

1. Anticorps bispécifique pour son utilisation dans le traitement d'une maladie cancéreuse, dans lequel ledit anticorps est administré en passant par la voie sous-cutanée, dans lequel l'anticorps représente un anticorps de redirection de lymphocytes T et comprend une spécificité qui est dirigée contre un antigène à la surface de lymphocytes T, une spécificité qui est dirigée contre un antigène associé à une tumeur et une région Fc.

2. Anticorps bispécifique pour son utilisation selon la revendication 1, **caractérisé en ce que** l'anticorps possède un format qui est analogue à celui d'une IgG.

3. Anticorps bispécifique pour son utilisation selon la revendication 1 ou 2, dans lequel l'antigène qui est associé à une tumeur est choisi parmi le groupe constitué par EpCAM, HER2/neu, HER3/neu, G250, CEA, MAGE, un protéoglycane, VEGF, EGFR, l'intégrine αᵥβ₃, HLA, HLA-DR, ASC, CD1, CD2, CD4, CD6, CD7, CD8, CD11, CD13, CD14, CD19, CD20, CD21, CD22, CD23, CD24, CD30, CD33, CD37, CD40, CD41, CD47, CD52, c-erb-2, CALLA, MHCII, CD44v3, CD44v6, p97, les gangliosides GM1, GM2, GM3, GD1a, GD1b, GD2, GD3, GT1b, GT3, GQ1, NY-ESO-1, NFX2, SSX2, SSX4 Trp2, gp100, la tyrosinase, Muc-1, la télomérase, la survivine, G250, p53, CA125 MUC, l'antigène Wue, l'antigène Lewis Y, HSP-27, HSP-70, HSP-72, HSP-90, Pgp, MCSP, EpHA2 et les cibles de surfaces cellulaires GC182, GT468 ou GT512, ou parmi le groupe constitué par EpCAM, HER2/neu, HER3/neu, G250, CEA, MAGE, un protéoglycane, VEGF, EGFR, l'intégrine αᵥβ₃, HLA, HLA-DR, ASC, CD1, CD2, CD4, CD6, CD7, CD8, CD11, CD13, CD14, CD19, CD20, CD21, CD22, CD23, CD24, CD30, CD33, CD37, CD40, CD41, CD47, CD52, c-erb-2, CALLA, MHCII, CD44v3, CD44v6, p97, les gangliosides GM1, GM2, GM3, GD1a, GD1b, GD2, GD3, GT1b, GT3, GQ1, NY-ESO-1, NFX2, SSX2, SSX4 Trp2, gp100, la tyrosinase, Muc-1, la télomérase, la survivine, G250, p53, CA125 MUC, l'antigène Wue, l'antigène Lewis Y, HSP-27, HSP-70, HSP-72, HSP-90, Pgp, MCSP, EpHA2 et les cibles de surfaces cellulaires GC182, GT468, PD-L1, la protéine E de l'arbovirus ou GT512.

4. Anticorps bispécifique pour son utilisation selon l'une quelconque des revendications 1 à 3, dans lequel l'antigène à la surface de lymphocytes T est choisi parmi le groupe constitué par CD3, CD2, CD4, CD5, CD6, CD8, CD28, CD40L et/ou CD44, de préférence CD3.

5. Anticorps bispécifique pour son utilisation selon l'une quelconque des revendications 1 à 4, dans lequel l'anticorps en question possède :
(i) une première spécificité dirigée contre l'antigène CD3 à la surface de lymphocytes T et une deuxième spécificité dirigée contre un antigène associé à une tumeur, qui est choisi parmi le groupe constitué par les gangliosides GM1, GM2, GM3, GD1a, GD1b, GD2, GD3, GT1b, GT3 et GQ1, ou qui est choisi parmi le groupe constitué par les antigènes tumoraux EpCAM, HER2/neu et CD20 ;
(ii) une première spécificité dirigée contre CD3 et une deuxième spécificité dirigée contre l'antigène GD2 associé à une tumeur ou l'antigène tumoral CD20 ou l'antigène tumoral EpCAM ou l'antigène HER2 associé à une tumeur ; de préférence dans lequel l'anticorps en question est le catumaxomab, FBTA05, l'Ektomun ou l'ertumaxomab ; ou
(iii) une première spécificité dirigée contre l'antigène CD3 à la surface de lymphocytes T et une deuxième spécificité dirigée contre un antigène associé à une tumeur, qui est choisi parmi le groupe constitué par GD3, CD19, CD30, CD33, PD-L1, EGF-R et la protéine E de l'arbovirus.

6. Anticorps bispécifique pour son utilisation selon l'une quelconque des revendications 1 à 5, dans lequel ladite maladie cancéreuse représente :
(a) une tumeur cancéreuse solide ou une tumeur cancéreuse non-solide, qui est de préférence choisie parmi le groupe constitué par un carcinome gastrique, un adénocarcinome, un mélanome, en particulier un mélanome malin, un carcinome du côlon, un carcinome du pancréas, un carcinome des ovaires, un cancer de l'utérus, un cancer du sein, un carcinome hépatocellulaire, un carcinome bronchique, des leucémies, de préférence des leucémies aigües ou chroniques, des lymphomes, de préférence le lymphome hodgkinien, le sarcome d'Ewing, un liposarcome, un fibrosarcome, un léiomyosarcome, un myélome, un thymome, ainsi que d'autres blastomes et sarcomes des tissus mous, en particulier un neuroblastome et un gliome ; ou
(b) un lymphome hodgkinien ou un lymphome non hodgkinien, par exemple un lymphone diffus à grandes cellules b.

7. Anticorps bispécifique pour son utilisation selon l'une quelconque des revendications 1 à 6, dans lequel l'anticorps en question est administré à un patient dont le diagnostic concerne au moins un type de cancer ; dans lequel le traitement comprend le fait de :
a. administrer par voie sous-cutanée une dose initiale dudit anticorps ; et par la suite
b. administrer par voie sous-cutanée au moins une dose de rappel dudit anticorps ; dans lequel ladite au moins une dose de rappel est de préférence inchangée par comparaison à la quantité de la dose précédente ou est supérieure à la quantité de la dose précédente ; de préférence dans lequel ladite au moins une dose de rappel excède la quantité administrée à titre de dose précédente à concurrence d'un facteur de 1,5 à 5, de manière plus préférée d'un facteur de 1,5 à 3, de manière encore plus préférée d'un facteur de 1,5 à 2,5.

8. Anticorps bispécifique pour son utilisation selon l'une quelconque des revendications 1 à 7, dans lequel l'anticorps en question est administré en combinaison avec :
(i) un médicament anticancéreux ou un anticorps anticancéreux, de préférence en recourant à une administration séparée ; et/ou
(ii) un glucocorticoïde ; dans lequel le glucocorticoïde est de préférence administré par voie locale au site de l'administration de l'anticorps.

9. Composition pharmaceutique pour son utilisation dans le traitement d'une maladie cancéreuse ; dans laquelle ladite composition pharmaceutique comprend un anticorps bispécifique, en particulier un anticorps trifonctionnel de redirection de lymphocytes T, tel que défini dans l'une quelconque des revendications 1 à 8 ; dans laquelle ladite composition est administrée par voie sous-cutanée.

10. Composition pharmaceutique pour son utilisation dans le traitement d'une maladie cancéreuse selon la revendication 9, dans laquelle la composition pharmaceutique comprend un tampon, de préférence un tampon d'acide organique, de manière plus préférée un tampon d'acide organique qui est choisi parmi le groupe constitué par un tampon de citrate, un tampon de phosphate, un tampon de succinate, un tampon de tartrate, de manière encore plus préférée un tampon de citrate ; dans laquelle la concentration du tampon, de préférence du tampon d'acide organique, de manière plus préférée du tampon de citrate, n'est de préférence pas supérieure à 100 mM, de manière plus préférée, pas supérieure à 50 mM, de manière encore plus préférée, pas supérieure à 40 mM, et de manière particulièrement préférée, pas supérieure à 30 mM, par exemple s'élève à 20 mM.

11. Polythérapie pour son utilisation dans le traitement d'une maladie cancéreuse, dans laquelle on administre l'anticorps bispécifique selon l'une quelconque des revendications 1 à 8 ou la composition pharmaceutique selon la revendication 9 ou 10 par voie sous-cutanée en combinaison avec au moins un médicament anticancéreux supplémentaire ou un anticorps anticancéreux supplémentaire (monoclonal, monospécifique), que l'on administre de préférence séparément par rapport à l'anticorps bispécifique.

12. Polythérapie pour son utilisation selon la revendication 11, dans laquelle ledit au moins un anticorps anticancéreux supplémentaire est choisi parmi le groupe constitué par le trastuzumab, l'alemtuzumab, le bévacizumab, le brentuximab-védotin, le cétuximab, le gemtuzumab, l'ozogamicine, l'ibritumomab tiuxétan, l'ipilmumab, le nimotuzumab, l'ofatumumab, le panitumumab, le rituximab et le tositumomab ; ou dans laquelle ledit au moins un anticorps anticancéreux supplémentaire est choisi parmi le groupe constitué par le nivolumab, le pembrolizumab et le blinatumomab.

13. Polythérapie pour son utilisation dans le traitement d'une maladie cancéreuse, dans laquelle on administre l'anticorps bispécifique selon l'une quelconque des revendications 1 à 8 ou la composition pharmaceutique selon la revendication 9 ou 10 par voie sous-cutanée en combinaison avec un glucocorticoïde ; dans laquelle le glucocorticoïde est de préférence administré par voie locale au site de l'administration de l'anticorps.
